# FASCICULE DE BREVET EUROPEEN

(11) **EP 2 174 644 B1**
(45) Date de publication et mention de la délivrance du brevet: **14.11.2012**
(21) Numéro de dépôt: 09168728.5
(22) Date de dépôt: 26.08.2009
(51) Int. Cl.: A61K 8/35, A61K 8/49, A61Q 17/04

(54) **Composition cosmétique contenant un dérivé de dibenzoylméthane et un composé dithiolane; procédé de photostabilisation du dérivé de dibenzoylméthane**
Kosmetische Zusammensetzung enthaltend ein Dibenzoylmethanderivat und ein Dithiolan; Verfahren zur Photostabilisierung des Dibenzoylmethanderivats
Cosmetic composition comprising a dibenzoylmethane derivative and a dithiolane; Photostabilization of a dibenzoylmethane derivative

(30) Priorité: 08.10.2008 FR 0856810
(43) Date de publication de la demande: 14.04.2010
(73) Titulaire: L'Oréal, 75008 Paris (FR)
(72) Inventeur: Lucet-Levannier, Karine, 92500, Reuil-Malmaison (FR); Muller, Benoit, 75010, Paris (FR)
(74) Mandataire: Miszputen, Laurent

(56) Documents cités:
- EP-A- 0 717 982
- FR-A- 2 908 769
- US-A- 6 013 663
- FUJI KAGAKU KOGYO K K: "Preparation and formulation of cyclic dithio derivatives as remedies for diabetic kidney diseases, hypoglycemic agents, hypolipidemic agents, and lenitives for digestive disorders" CA HOST- CA, 1998, XP002524712

## Description

La présente invention est relative à une composition cosmétique contenant l'association d'au moins un filtre du type dérivé du dibenzoylméthane et d'au moins un composé dithiolane de formule (I) dont on donnera la définition ci-après.

Elle concerne également un procédé de photostabilisation vis-à-vis du rayonnement d'au moins un filtre du type dérivé du dibenzoylméthane par un composé dithiolane de formule (I) particulier de formule (I) dont on donnera la définition ci-après.

La présente invention est relative également à l'utilisation d'au moins un composé dithiolane de formule (I) particulier de formule (I) dans une composition comprenant dans un support cosmétiquement acceptable, au moins un dérivé du dibenzoylméthane dans le but de d'améliorer l'efficacité de ladite composition vis-à-vis des rayons UV-A.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

Dans le but d'assurer une protection de la peau et des matières kératiniques contre le rayonnement UV, on utilise généralement des compositions antisolaires comprenant des filtres organiques, actifs dans l'UV-A et actifs dans l'UV-B. La majorité de ces filtres est liposoluble.

A cet égard, une famille de filtres UV-A particulièrement intéressante est actuellement constituée par les dérivés du dibenzoylméthane, et notamment le 4-ter-butyl-4'-méthoxydibenzoyl méthane, qui présentent en effet un fort pouvoir d'absorption intrinsèque. Ces dérivés du dibenzoylméthane, qui sont maintenant des produits bien connus en soi à titre de filtres actifs dans les UV-A, sont notamment décrits dans les demandes de brevets français FR-A-2326405 et

FR-A-2440933, ainsi que dans la demande de brevet européen EP-A-0114607 ; le 4-ter-butyl- 4'-méthoxydibenzoyl méthane est par ailleurs actuellement proposé à la vente sous la dénomination commerciale de « PARSOL 1789 ® » par la Société DSM NUTRITIONAL PRODUCTS.

Malheureusement, il se trouve que les dérivés du dibenzoylméthane sont des produits relativement sensibles au rayonnement ultraviolet (surtout UV-A), c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV.

La photostabilisation des dérivés du dibenzoylméthane vis-à-vis du rayonnement UV par des composés amidés constitue donc, à ce jour, un problème qui n'a pas encore été résolu de manière complètement satisfaisante.

Or, la Demanderesse vient maintenant de découvrir, de façon surprenante, qu'en associant aux dérivés du dibenzoylméthane mentionnés ci-dessus un composé dithiolane de formule (I) particulière que l'on définira plus loin en détail, il était possible d'améliorer encore de manière substantielle et remarquable, la stabilité photochimique (ou photostabilité) de ces mêmes dérivés du dibenzoylméthane et leur efficacité dans l'UV-A. Les compositions contenant une telle association conduisent également après application à une répartition plus homogène du filtre dibenzoylméthane.

Cette découverte est à la base de la présente invention.

Ainsi, conformément à l'un des objets de la présente invention, il est maintenant proposé une composition comprenant dans un support cosmétiquement acceptable au moins un système filtrant UV, caractérisée par le fait qu'elle comprend :
(a) au moins un dérivé du dibenzoylméthane et
(b) au moins un composé dithiolane de formule (I) dont on donnera la définition ci-après.

Un autre objet de l'invention concerne également un procédé pour améliorer la stabilité chimique vis-à-vis du rayonnement UV d'au moins un dérivé du dibenzoylméthane consistant à associer audit dérivé de dibenzoylméthane une quantité efficace d'au moins un composé dithiolane de formule (I) dont on donnera les définitions ci-après

La présente invention a également pour objet l'utilisation d'au moins un composé dithiolane de formule (I) dans une composition comprenant dans un support cosmétiquement acceptable, au moins un dérivé du dibenzoylméthane dans le but d'améliorer l'efficacité de ladite composition vis-à-vis des rayons UV-A.

D'autres caractéristiques, aspects et avantages de l'invention apparaîtront à la lecture de la description détaillée qui va suivre.

Par "cosmétiquement acceptable", on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Par « quantité efficace », on entend une quantité suffisante pour obtenir une amélioration notable et significative de la photostabilité du ou des dérivés du dibenzoylméthane dans la composition cosmétique. Cette quantité minimale en composé dithiolane de formule (I), qui peut varier selon la nature du support retenu pour la composition, peut être déterminée sans aucune difficulté au moyen d'un test classique de mesure de photostabilité, tel que celui donné dans les exemples ci-après.

Les composés dithiolanes conformes à la présente invention répondent à la formule (I) suivante : 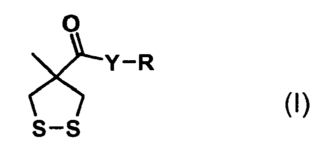 dans laquelle :
Y désigne O ou NR₁
R₁ désigne un atome d'hydrogène ; un groupe hydrocarboné alkyle saturé linéaire en C₁-C₂₀ ou ramifié en C₃-C₂₀ ou insaturé en C₂-C₂₀ ; un groupe aryle éventuellement substitué par un ou plusieurs hydroxyles et/ou par un ou plusieurs radicaux alkoxy en C₁-C₈ ;
R désigne un atome d'hydrogène ; un groupe hydrocarboné alkyle saturé linéaire en C₁-C₂₀ ou ramifié en C₃-C₂₀ ou insaturé en C₂-C₂₀ ou un groupe aryle ou un groupe alkyle saturé en C₁-C₈ possédant un substituant aryle éventuellement substitué par un ou plusieurs hydroxyles et/ou par un ou plusieurs radicaux alcoxy en C₁-C_{8 ;}
R possède éventuellement un ou plusieurs substituants choisis parmi OR₂, SR₂, NR₂R₃, COOR₂ dans lesquels :
   R₂ désigne un atome d'hydrogène ou un groupe hydrocarboné saturé linéaire en C₁-C₅ ou ramifié en C₃-C₅ ou insaturé en C₂-C₅, ou un groupe aryle
   R₃ désigne un atome d'hydrogène ; un groupe hydrocarboné saturé linéaire en C₁-C₅ ou ramifié en C₃-C₅ ou insaturé en C₂-C₅ un groupe aryle ; un groupe acétyle.

R et R1 peuvent former un cycle choisi parmi pyrrolidine, pyrroline, pipéridine, pipérazine, morpholine, thiomorpholine, azépine ainsi que leurs sels, leurs chélates, leurs solvates et leurs isomères optiques.

Les sels des composés décrits dans la présente invention comprennent des sels non toxiques conventionnels desdits composés tels que ceux formés à partir d'acides organiques ou minéraux. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Les sels de bases organiques ou minérales telles que les sels de triéthanolamine, d'aminopropanediol, de sodium, de zinc.

Les solvates acceptables pour l'usage non thérapeutique des composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

Les isomères optiques sont notamment les énantiomères et les diastéréoismères.

De façon préférentielle, les groupes aryle désignent phényle ou benzyle.

De façon préférentielle, les groupes alcoxy sont linéaires en C₁-C₄ et plus préférentiellement méthoxy, éthoxy, propoxy, butoxy et encore plus préférentiellement méthoxy.

De façon préférentielle, les groupes hydrocarbonés sont des alkyles linéaires ou ramifiés et peuvent être choisis parmi : méthyle, éthyle, propyle, isopropyle, butyle, isobutyle, tertio-butyle pentyle, hexyle, heptyle octyle, nonyle, decyle, undecyle, docecyle, tridecyle, tetradecyle, pentadecyle, hexadecyle, heptadecyle, octadecyle, nonadecyle, icosyle.

De façon plus préférentielle, les groupes hydrocarbonés sont des radicaux alkyles linéaires ou ramifiés saturés en C₁-C₈ : méthyle, éthyle propyle isopropyle butyle isobutyle, tertio-butyle, pentyle, hexyle, heptyle, octyle.

De façon encore plus préférentielle lorsque Y désigne NR₁ alors R₁ = H

De façon très préférentielle, R = H ou un radical alkyle en C₁-C₈.

Parmi les composés de formule (I), on utilisera plus particulièrement les produits suivants :

| **N°** | **Structure** | **Nom Chimique** |
|---|---|---|
| 1 | 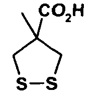 | Acide 4-methyl-1,2-dithiolane-4-carboxylique |
| 2 | 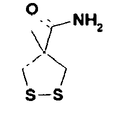 | 4-methyl-1,2-dithiolane-4-carboxamide |
| 3 | 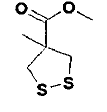 | Méthyl 4-methyl-1,2-dithiolane-4-carboxylate |
| 4 | 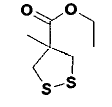 | Ethyl 4-methyl-1,2-dithiolane-4-carboxylate |
| 5 | 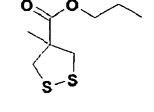 | Propyl 4-methyl-1,2-dithiolane-4-carboxylate |
| 6 | 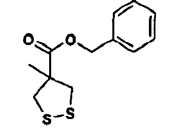 | Benzyl 4-methyl-1,2-dithiolane-4-carboxylate |
| 7 | 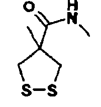 | N-methyl 4-methyl-1,2-dithiolane-4-carboxamide |
| 8 | 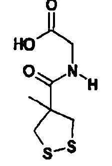 | {[(4-methyl-1,2-dithiolan-4-yl)carbonyl]amino} acetic acide |
| 9 | 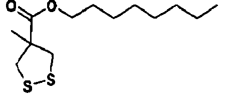 | Octyl 4-methyl-1,2-dithiolane-4-carboxylate |
| 10 | 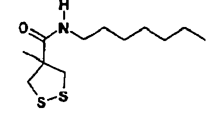 | N-heptyl-4-methyl-1,2-dithiolane-4-carboxamide |
| 11 | 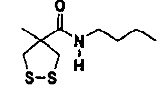 | N-butyl-4-methyl-1,2-dithiolane-4-carboxamide |
| 12 | 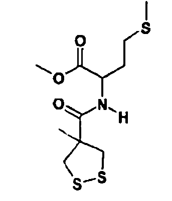 | Methyl 2-{[(4-methyl-1,2-dithiolan-4-yl)carbonyl]amino}-4-(methylsulfanyl)butanoate |
| 14 | 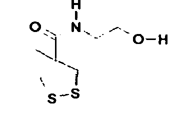 | N-(2-hydroxyethyl)-4-methyl-1,2-dithiolane-4-carboxamide |
| 15 | 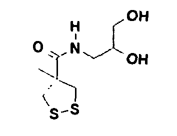 | N-(2,3-dihydroxypropyl)-4-methyl-1,2-dithiolane-4-carboxamide |
| 16 | 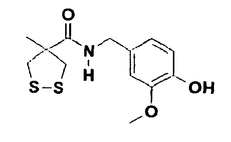 | N-(4-hydroxy-3-methoxybenzyl)-4-methyl-1,2-dithiolane-4-carboxamide |
| 17 | 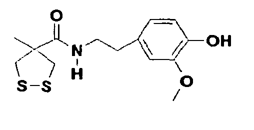 | N-[2-(4-hydroxy-3-methoxyphenyl)ethyl]-4-methyl-1,2-dithiolane-4-carboxamide |
| 18 | 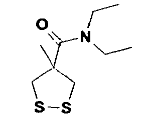 | N,N-diethyl-4-methyl-1,2-dithiolane-4-carboxamide |
| 19 | 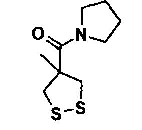 | [(4-methyl-1,2-dithiolan-4-yl)carbonyl]pyrrolidine |
| 20 | 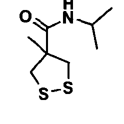 | 4-methyl-N-(1-methylethyl)-1,2-dithiolane-4-carboxamide |
| 21 | 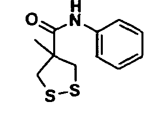 | 4-methyl-N-phenyl-1,2-dithiolane-4-carboxamide |
| 22 | 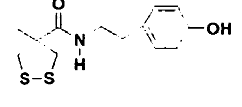 | N-[2-(4-hydroxyphenyl)ethyl]-4-methyl-1,2-dithiolane-4-carboxamide |
| 23 | 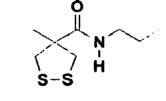 | N-propyl-4-methyl-1,2-dithiolane-4-carboxamide |
| 24 | 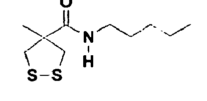 | N-pentyl-4-methyl-1,2-dithiolane-4-carboxamide |
| 25 | 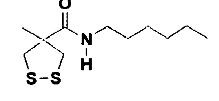 | N-hexyl-4-methyl-1,2-dithiolane-4-carboxamide |

Parmi ces composés, on préfère plus particulièrement les composés suivants

| **N°** | **Structure** | **Nom Chimique** |
|---|---|---|
| 1 | 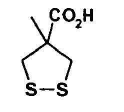 | Acide 4-methyl-1,2-dithiolane-4-carboxylique |
| 2 | 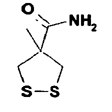 | 4-methyl-1,2-dithiolane-4-carboxamide |
| 9 | 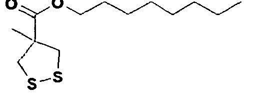 | Octyl 4-methyl-1,2-dithiolane-4-carboxylate |
| 10 | 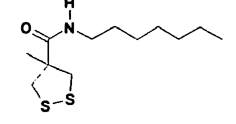 | N-heptyl-4-methyl-1,2-dithiolane-4-carboxamide |

### Synthèse

Les composés de formule (I) peuvent être obtenus suivant les voies décrites ci-dessous et documentées dans la revue de Lene Teuber, Sulfur reports, 9(4), 257-349,1990 Naturally occuring 1,2-dithiolanes and 1,2,3-tritianes. Chemical and Biological Properties et dans la demande de brevet EP 0 869 126 A1.

A partir de l'acide 2,2 bis(hydroxymethyl)propionique (CAS : 4767-03-7), par fonctionnalisation des hydroxyles en groupements partants X (alkyle ou aryles sulfonates tels que mésylates ou tosylates ou halogènes tels que l'iode, le brome ou le chlore) suivie de l'introduction du soufre selon le schéma réactionnel suivant :

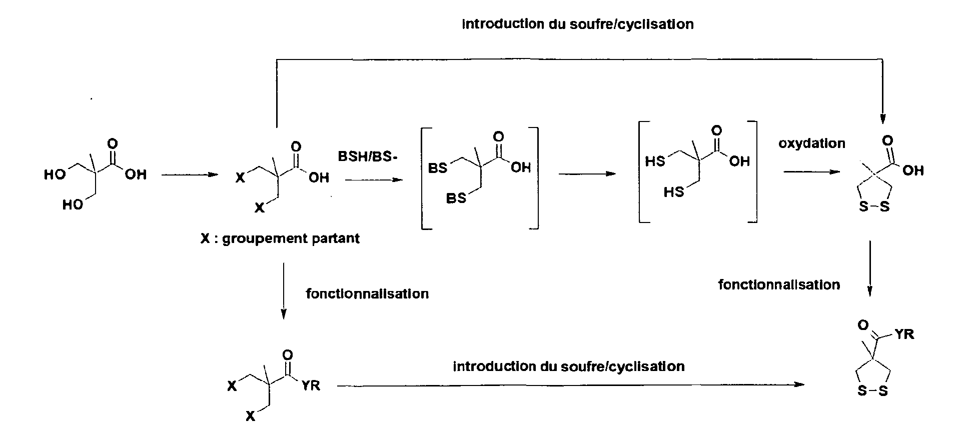

Cette introduction du souffre peut s'effectuer :
(i) en une étape à l'aide de disulfure de métaux (comme le Na2S2) ou des sels de tétrathiomolybdates dans des solvants polaires protiques ou aprotiques (par exemple eau, DMF, méthanol, acétonitrile) pour conduire au dithiolane.
(ii) ou en deux étapes en formant un intermédiaire di-thiol, qui en présence d'un oxydant (oxygène, DMSO, FeCl₃, I₂, Br₂, iodure de sodium, trifluoroacetates de thallium, triflates d'argent, eau oxygénée, iodate et periodate de sodium, hypochlorite de sodium, ferricyanide de potassium, oxyde de chrome), en milieu neutre ou basique conduit à la formation du dithiolane. Dans ce cas, le di-thiol est obtenu par transformation (en milieu basique ou acide) dans un solvant polaire ou apolaire d'une espèce intermédiaire via des dérivés de l'acide thioacétique CH3COSH (en présence de base), par de la thiourée ou du NaSH, par la formation de dithiosulfoantes (sels de Bunte).

La fonctionnalisation de l'acide carboxylique COOH en fonction COYR peut s'effectuer suivant les méthodes classiques d'activation des acides (décrites dans Comprehensive Organic Transformation de R. LAROCK Wiley VCH Ed. dans le chapitre Interconversion of nitriles, carboxylic acids and derivatives). De préférence les méthodes utilisées privilégient le passage au chlorure d'acide (par utilisation de chlorure de thionyle ou d'oxalyle, ou de 1-chloro-n.n.2-trimethyl-1-propenamine) ou par la formation d'anhydride mixte (à l'aide de chloroformiate d'alkyles) ou l'utilisation de carbodiimides ou de diéthylcyanophosphate (Phosphorus in organic synthesis-Xl, Amino acids and peptides-XXI, Reaction of diethyl phosphorocyanidate with carboxylic acids. A new synthesis of carboxylic esters and amides, Tetrahedron, 32, 1976, 2211-2217).*

Les solvants utilisées peuvent être polaires ou apolaires, protiques ou aprotiques (par exemple toluène, dichlorométhane, THF, DMF, acétonitrile, eau, méthanol, isopropanol)

Toutes ces réactions peuvent s'effectuer à des températures comprises entre - 20 et 100°C.

Avantageusement, le composé 1 peut être obtenu suivant la voie décrite ci-dessous à partir de l'acide dichloro-pivalique suivant un procédé en un pot, finalisé par un précipitation.

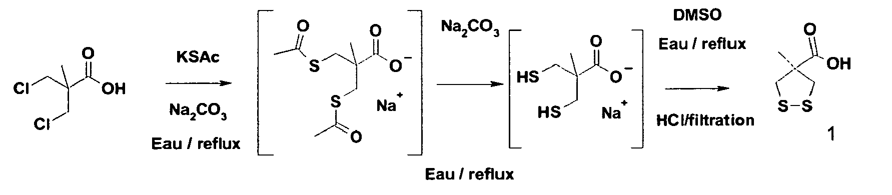

Avantageusement, le composé 2 peut être obtenu à partir du composé 1, préférentiellement à l'aide de chloroformiate d'isobutyle ou de chlorure d'oxallyle.

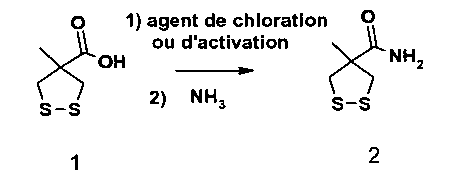

Les composés dithiolanes de formule (I) sont de préférence présents dans la composition selon l'invention en une teneur allant de 0,1% à 40% en poids, par rapport au poids total de la composition, et de préférence allant de 0,1 à 30 % en poids.

Parmi les dérivés du dibenzoylméthane, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert-butyl-4'-méthxydibenzoylméthane,
- le 2,4-diméthyl-4'-méthoxydibenzoylméthane,
- le 2,6-diméthyl-4-tert-butyl-4'-méthoxydibenzoylméthane.

Parmi les dérivés du dibenzoylméthane mentionnés ci-dessus, on utilisera en particulier le 4-isopropyl-dibenzoylméthane, vendu sous la dénomination de "EUSOLEX 8020" par la Société MERCK, et répondant à la formule suivante :

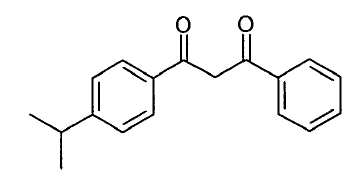

On préfère tout particulièrement mettre en oeuvre le 4-(ter.-butyl) 4'-méthoxy dibenzoylméthane ou Butyl Methoxy Dibenzoylmethane, proposé à la vente sous la dénomination commerciale de "PARSOL 1789" par la Société DSM NUTRITIONAL PRODUCTS ; ce filtre répond à la formule suivante :

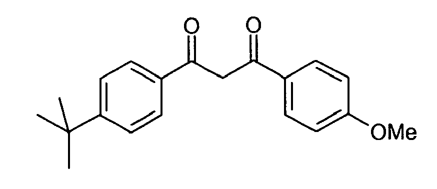

Le ou les dérivés du dibenzoylméthane peuvent être présents dans les compositions conformes à l'invention à des teneurs qui varient de préférence de 0,01 à 10% en poids et plus préférentiellement de 0,1 à 6% en poids par rapport au poids total de la composition.

Les compositions conformes à l'invention peuvent comporter en plus d'autres filtres UV organiques ou inorganiques complémentaires actifs dans l'UVA et/ou l'UVB hydrosolubles ou liposolubles ou bien insolubles dans les solvants cosmétiques couramment utilisés.

Bien entendu, l'homme de l'art veillera à choisir le ou les éventuels filtres complémentaires et/ou leurs quantités de manière telle que les propriétés avantageuses attachées intrinsèquement aux compositions conformes à l'invention ne soient pas, ou substantiellement pas, altérées par la ou les adjonctions envisagées notamment l'amélioration de la photostabilité du dérivé de dibenzoylméthane.

Les filtres organiques complémentaires sont notamment choisis parmi les anthranilates ; les dérivés cinnamiques ; les dérivés salicyliques, les dérivés du camphre ; les dérivés de la benzophénone ; les dérivés de β , β - diphénylacrylate ; les dérivés de triazine ; les dérivés de benzotriazole ; les dérivés de benzalmalonate notamment ceux cités dans le brevet US5624663 ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 ; les dérivés de mérocyanines tels que ceux décrits dans les demandes WO04006878, WO05058269 et WO06032741 et leurs mélanges .

Comme exemples d'agents photoprotecteurs organiques complémentaires, on peut citer ceux désignés ci-dessous sous leur nom INCl :

### Dérivés cinnamiques :

Ethylhexyl Methoxycinnamate vendu notamment sous le nom commercial PARSOL MCX par DSM Nutritional Products, Inc,, Isopropyl Methoxycinnamate Isoamyl Methoxycinnamate vendu sous le nom commercial NEO HELIOPAN E 1000 par SYMRISE,
DEA Methoxycinnamate, Diisopropyl Methylcinnamate,
Glyceryl Ethylhexanoate Dimethoxycinnamate

Dérivés de l'acide para-aminobenzoique :
PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par SYMRISE,
Dipropyleneglycol Salicylate vendu sous le nom « DIPSAL » par SCHER,
TEA Salicylate, vendu sous le nom « NEO HELIOPAN TS » par SYMRISE,

### Dérivés de β,β-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF, Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF, Benzophenone-12
2-(4-diethylamino-2-hydroxybenzoyl)-benzoate de n-hexyle vendu sous le nom commercial « UVINUL A+ » par BASF.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial « NEO HELIOPAN AP » par SYMRISE,

### Dérivés du phenyl benzotriazole :

Drometrizole Trisiloxane vendu sous le nom « Silatrizole » par RHODIA CHIMIE Methylène bis-Benzotriazolyl Tetramethylbutylphénol, vendu sous forme solide sous le nom commercial « MIXXIM BB/100 » par FAIRMOUNT CHEMICAL ou sous forme micronisée en dispersion aqueuse sous le nom commercial « TINOSORB M » par CIBA SPECIALTY CHEMICALS,

### Dérivés de triazine :

- Bis-Ethylhexyloxyphenol Methoxyphenyl Triazine vendu sous le nom commercial «TINOSORB S » par CIBA GEIGY,
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4bis (4'-aminobenzoate de n-butyle)-6-(aminopropyltrisiloxane)-s-triazine,
- la 2,4,6-tris(4'-amino benzalmalonate de dinéopentyle)-s-triazine
- la 2,4,6-tris-(4'-amino benzalmalonate de diisobutyle)-s- triazine,
- la 2,4bis (4'-aminobenzoate de n-butyle)-6-(aminopropyltrisiloxane)-s-triazine,
- la 2,4bis (4'-aminobenzalmalonate de dinéopentyle)-6-(4'-aminobenzoate de n-butyle)-s-triazine,
- les filtres triazines symétriques décrits dans le brevet US6,225,467, la demande WO2004/085412 (voir composés 6 et 9) ou le document « Symetrical Triazine Derivatives » IP.COM Journal , IP.COM INC WEST HENRIETTA, NY, US (20 septembre 2004) notamment les 2,4,6-tris-(biphényl)-1,3,5-triazines (en particulier la 2,4,6-tris(biphenyl-4-yl-1,3,5-triazine et la 2,4,6-tris(terphenyl)-1,3,5-triazine qui est repris dans les demandes de brevet WO06/035000, WO06/034982, WO06/034991, WO06/035007, WO2006/034992, WO2006/034985.

### Dérivés anthraniliques :

Menthyl anthranilate vendu sous le nom commercial commercial « NEO HELIOPAN MA » par SYMRISE,

### Dérivés d'imidazolines :

Ethylhexyl Dimethoxybenzylidene Dioxoimidazoline Propionate,

### Dérivés du benzalmalonate :

Polyorganosiloxane à fonctions benzalmalonate comme le Polysilicone-15 vendu sous la dénomination commerciale « PARSOL SLX » par DSM Nutritional Products, Inc

### Dérivés de 4,4-diarylbutadiène :

- 1,1-dicarboxy (2,2'-diméthyl-propyl)-4,4-diphénylbutadiène

### Dérivés de benzoxazole :

2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V et leurs mélanges.

### Dérivés de mérocyanine

Octyl-5-N,N-diethylamino-2-phenysulfonyl-2,4-pentadienoate

Les filtres organiques préférentiels sont choisis parmi
Ethylhexyl Methoxycinnamate
Ethylhexyl Salicylate,
Homosalate,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Bis-Ethylhexyloxyphenol Methoxyphenyl Triazine
Ethylhexyl triazone,
Diethylhexyl Butamido Triazone,
la 2,4bis (4'-aminobenzoate de n-butyle)-6-(aminopropyltrisiloxane)-s-triazine,
la 2,4,6-tris(4'-amino benzalmalonate de dinéopentyle)-s-triazine
la 2,4,6-tris-(4'-amino benzalmalonate de diisobutyle)-s- triazine
la 2,4bis (4'-aminobenzalmalonate de dinéopentyle)-6-(4'-aminobenzoate de n-butyle)-s-triazine,
la 2,4,6-tris(biphenyl-4-yl-1,3,5-triazine
la 2,4,6-tris(terphenyl)-1,3,5-triazine
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
Octyl-5-N,N-diethylamino-2-phenysulfonyl-2,4-pentadienoate et leurs mélanges.

Les filtres inorganiques complémentaires sont choisis parmi des pigments d'oxydes métalliques enrobés ou non dont la taille moyenne des particules primaires: est préférentiellement comprise entre 5 nm et 100 nm (de préférence entre 10 nm et 50 nm) comme par exemple des pigments d'oxyde de titane (amorphe ou cristallisé sous forme rutile et/ou anatase), de fer, de zinc, de zirconium ou de cérium ou leurs mélanges qui sont tous des agents photoprotecteurs UV bien connus en soi.

Les pigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

De façon connue, les silicones sont des polymères ou oligomères organo-siliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium.

Le terme "silicones" englobe également les silanes nécessaires à leur préparation, en particulier, les alkyl silanes.

Les silicones utilisées pour l'enrobage des pigments convenant à la présente invention sont de préférence choisies dans le groupe contenant les alkyl silanes, les polydialkylsiloxanes, et les polyalkylhydrogénosiloxanes. Plus préférentiellement encore, les silicones sont choisies dans le groupe contenant l'octyl triméthyl silane, les polydiméthylsiloxanes et les polyméthylhydrogénosiloxanes.

Bien entendu, les pigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Les pigments enrobés sont plus particulièrement des oxydes de titane enrobés :
- de silice tels que le produit "SUNVEIL" de la société IKEDA et le produit " Eusolex T-AVO" de la société MERCK
- de silice et d'oxyde de fer tels que le produit "SUNVEIL F" de la société IKEDA,
- de silice et d'alumine tels que les produits "MICROTITANIUM DIOXIDE MT 500 SA" et "MICROTITANIUM DIOXIDE MT 100 SA" de la société TAYCA, "TIOVEIL" de la société TIOXIDE, et « Mirasun TiW 60 » de la société Rhodia,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que le produit "MICROTITANIUM DIOXIDE MT 100 TV, MT 100 TX, MT 100 Z, MT-01 de la société TAYCA, les produits "Solaveil CT-10 W", "Solaveil CT 100" et "Solaveil CT 200" de la société UNIQEMA,
- de silice, d'alumine et d'acide alginique tel que le produit " MT-100 AQ" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS"de la société TAYCA,
- de silice, d'alumine, de stéarate d'aluminium et traités par une silicone tels que le produit "STT-30-DS" de la société TITAN KOGYO,
- de silice et traité par une silicone tel que le produit "UV-TITAN X 195" de la société KEMIRA, ou le produit SMT-100 WRS de la société TAYCA.
- d'alumine et traités par une silicone tels que les produits "TIPAQUE TTO-55 (S)" de la société ISHIHARA, ou "UV TITAN M 262" de la société KEMIRA, de triéthanolamine tels que le produit "STT-65-S" de la société TITAN KOGYO,
- d'acide stéarique tels que le produit "TIPAQUE TTO-55 (C)" de la société ISHIHARA,
- d'hexamétaphosphate de sodium tels que le produit "MICROTITANIUM DIOXIDE MT 150 W" de la société TAYCA.

D'autres pigments d'oxyde de titane traités avec une silicone sont de préférence le TiO2 traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO2 traité par un polydiméthylsiloxane et dont la taille moyenne des particules élémentaires est de 21 nm tel que celui vendu sous la dénomination commerciale "70250 Cardre UF TiO2S13" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les pigments d'oxyde de zinc non enrobés, sont par exemple
- ceux commercialisés sous la dénomination "Z-cote" par la société Sunsmart ;
- ceux commercialisés sous la dénomination "Nanox" par la société Elementis ;
- ceux commercialisés sous la dénomination "Nanogard WCD 2025" par la société Nanophase Technologies ;

Les pigments d'oxyde de zinc enrobés sont par exemple
- ceux commercialisés sous la dénomination « Z-COTE HP1 » par la société SUNSMART (ZnO enrobé dimethicone) ;
- ceux commercialisés sous la dénomination "Oxide zinc CS-5" par la société Toshibi (ZnO enrobé par polymethylhydrogenesiloxane) ;
- ceux commercialisés sous la dénomination "Nanogard Zinc Oxide FN" par la société Nanophase Technologies (en dispersion à 40% dans le Finsolv TN, benzoate d'alcools en C12-C15) ;
- ceux commercialisés sous la dénomination "DAITOPERSION ZN-30" et "DAITOPERSION Zn-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD Ultrafine ZnO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "Escalol Z100" par la société ISP (ZnO traité alumine et dispersé dans le mélange methoxycinnamate d'ethylhexyle / copolymère PVP-hexadecene / methicone) ;
- ceux commercialisés sous la dénomination "Fuji ZnO-SMS-10" par la société Fuji Pigment (ZnO enrobé silice et polymethylsilsesquioxane) ;
- ceux commercialisés sous la dénomination "Nanox Gel TN" par la société Elementis (ZnO dispersé à 55% dans du benzoate d'alcools en C12-C15 avec polycondensat d'acide hydroxystéarique).

Les pigments d'oxyde de cérium non enrobé sont vendus par exemple sous la dénomination "COLLOIDAL CERIUM OXIDE" par la société RHONE POULENC.

Les nanopigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les pigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Les filtres UV additionnels sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions aqueuses conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire autre que les cires apolaires telles que définies précédemment ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les amides grasses (comme l'isopropyl lauroyl sarcosinate vendu sous la dénomination d' « Eldew SL-205 » par la société Ajinomoto), les acides ou les esters gras comme le benzoate d'alcools en C12-C15 vendu sous la dénomination commerciale « Finsolv TN » ou « Witconol TN » par la société WITCO, le Benzoate de 2-éthylphenyle comme le produit commercial vendu sous le nom X-TEND 226 ® par la société ISP, le palmitate d'octyle, le lanolate d'isopropyle, les triglycérides dont ceux des acides caprique/caprylique, le dicaprylyl carbonate vendu sous la dénomination « Cetiol CC » par la société Cognis), les esters et éthers gras oxyéthylénés ou oxypropylénés; les huiles siliconées (cyclométhicone, polydiméthysiloxanes ou PDMS) ou fluorées, les polyalkylènes, les trimellitates de trialkyle comme le trimellitate de tridécyle.

Comme composés cireux, on peut citer la cire de carnauba, la cire d'abeille, l'huile de ricin hydrogénée, les cires de polyéthylène et les cires de polyméthylène comme celle vendue sous la dénomination Cirebelle 303 par la société SASOL.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Comme épaississants hydrophiles, on peut citer les polymères carboxyvinyliques tels que les Carbopols (Carbomers) et les Pemulen (Copolymère acrylate/C₁₀-C₃₀-alkylacrylate) ; les polyacrylamides comme par exemple les copolymères réticulés vendus sous les noms Sepigel 305 (nom C.T.F.A. : polyacrylamide/C₁₃-₁₄ isoparaffin/Laureth 7) ou Simulgel 600 (nom C.T.F.A. : acrylamide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryloyldimethyl taurate ou le SIMULGEL 800 commercialisé par la société SEPPIC (nom CTFA : sodium polyacryolyldimethyl taurate / polysorbate 80 / sorbitan oleate) ; les copolymères d'acide 2-acrylamido 2-méthylpropane sulfonique et d'hydroxyethyl acrylate comme le SIMULGEL NS et le SEPINOV EMT 10 commercialisés par la société SEPPIC ; les dérivés cellulosiques tels que l'hydroxyéthylcellulose , les polysaccharides et notamment les gommes telles que la gomme de Xanthane ; et leurs mélanges.

Comme épaississants lipophiles, on peut citer les polymères synthétiques tels que les poly C₁₀-C₃₀ alkyl acrylates vendu sous la dénomination « INTELIMER IPA 13-1 » et « INTELIMER IPA 13-6 » par la société Landec ou encore les argiles modifiées telles que l'hectorite et ses dérivés, comme les produits commercialisés sous les noms de Bentone.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées, notamment l'amélioration de la photostabilité du dérivé de dibenzoylméthane.

Les compositions selon l'invention peuvent être préparées selon les techniques bien connues de l'homme de l'art. Elles peuvent se présenter en particulier sous forme d'émulsion, simple ou complexe (H/E, E/H, H/E/H ou E/H/E) telle qu'une crème, un lait ou d'un gel crème ; sous la forme d'un gel aqueux ; sous la forme d'une lotion. Elles peuvent éventuellement être conditionnées en aérosol et se présenter sous forme de mousse ou de spray.

De préférence, les compositions selon l'invention se présentent sous la forme d'une émulsion huile-dans-eau ou eau-dans huile.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E). Les émulsions peuvent contenir également d'autres types de stabilisants comme par exemple des charges, des polymères gélifiants ou épaississants.

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les diméthicone copolyols tels que le mélange de cyclométhicone et de diméthicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90R par la société Goldschmidt et le mélange de cétyl diméthicone copolyol, d'isostéarate de polyglycérole (4 moles) et de laurate d'hexyle vendu sous la dénomination ABIL WE 09 par la société Goldschmidt. On peut y ajouter aussi un ou plusieurs co-émulsionnants, qui, de manière avantageuse, peuvent être choisis dans le groupe comprenant les esters alkylés de polyol.

Comme esters alkylés de polyol, on peut citer notamment les esters de polyéthylèneglycol comme le PEG-30 Dipolyhydroxystearate tel que le produit commercialisé sous le nom Arlacel P135 par la socité ICl ; Comme esters de glycérol et/ou de sorbitan, on peut citer par exemple l'isostéarate de polyglycérol, tel que le produit commercialisé sous la dénomination lsolan Gl 34 par la société Goldschmidt ; l'isostéarate de sorbitan, tel que le produit commercialisé sous la dénomination Arlacel 987 par la société ICl ; l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) comme le mélange PEG-100 Stearate/ Glyceryl Stearate commercialisé par exemple par la société ICI sous la dénomination Arlacel 165 ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition auto-émulsionnante, comme décrit par exemple dans le document WO-A-92/06778.

Parmi les autres stabilisants d'émulsion, on utilisera plus particulièrement les polymères d'acide isophtalique ou d'acide sulfoisophtalique, et en particulier les copolymères de phtalate / sulfoisophtalate / glycol par exemple le copolymère de Diéthylèneglycol / Phtalate / lsophtalate / 1,4-cyclohexane-diméthanol (nom INCl : Polyester-5) vendu sous les dénominations "Eastman AQ polymer" (AQ35S, AQ38S, AQ55S, AQ48 Ultra) par la société Eastman Chemical.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin, des produits de protection solaire et des produits de maquillage.

Les compositions cosmétiques selon l'invention peuvent par exemple être utilisées comme produit de maquillage.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Les compositions selon l'invention peuvent également comprendre en plus des actifs additionnels cosmétiques et dermatologiques.

Les actifs additionnels pourront notamment être choisis parmi les agents hydratants, les agents desquamants, les agents améliorant la fonction barrière, les agents dépigmentants, les agents antioxydants, les agents dermodécontractants, les agents anti-glycation, les agents stimulant la synthèse de macromolécules dermiques et/ou épidermiques et/ou empêchant leur dégradation, les agents stimulant la prolifération des fibroblastes ou des kératinocytes et/ou la différenciation des kératinocytes, les agents favorisant la maturation de l'enveloppe cornée, les inhibiteurs de NO-synthases, les antagonistes des récepteurs périphériques des benzodiazépines (PBR), les agents augmentant l'activité de la glande sébacée, les agents stimulant le métabolisme énergétique des cellules, les agents tenseurs, les agents liporestructurants, les agents amincissants, les agents favorisant la microcirculation cutanée, les agents apaisants et/ou anti-irritants, les sébo-régulateurs ou anti-séborrhéiques, les agents astringents, les agents cicatrisants, les agents anti-inflammatoires, et les agents anti-acné.

L'homme du métier choisira le ou lesdits actifs en fonction de l'effet recherché sur la peau, les cheveux, les cils, les sourcils, les ongles.

Pour le soin et/ou le maquillage des peaux âgées, il choisira de préférence au moins un actif choisi parmi les agents hydratants, les agents desquamants, les agents améliorant la fonction barrière, les agents dépigmentants, les agents antioxydants, les agents dermodécontractants, les agents anti-glycation, les agents stimulant la synthèse de macromolécules dermiques et/ou épidermiques et/ou empêchant leur dégradation, les agents stimulant la prolifération des fibroblastes ou des kératinocytes et/ou la différenciation des kératinocytes, les agents favorisant la maturation de l'enveloppe cornée, les inhibiteurs de NO-synthases, les antagonistes des récepteurs périphériques des benzodiazépines (PBR), les agents augmentant l'activité de la glande sébacée, les agents stimulant le métabolisme énergétique des cellules, , les agents liporestructurants et les agents favorisant la microcirculation cutanée pour le contour des yeux.

La composition pourra comprendre en outre au moins un ingrédient tel que des charges à effet flouteur ou des agents favorisant la coloration naturelle de la peau, destiné à compléter l'effet biologique de ces actifs ou apporter un effet anti-âge immédiat visuel.

Pour le soin et/ou le maquillage des peaux grasses, l'homme du métier choisira de préférence au moins un actif choisi parmi les agents desquamants, agents sébo-régulateurs ou anti-séborrhéiques, les agents astringents.

La composition pourra comprendre en outre au moins un ingrédient additionnel destiné à compléter l'effet biologique de ces actifs ou apporter un effet immédiat visuel ; on peut citer notamment les agents matifiants, les charges à effet flouteur, les agents fluorescents, les agents favorisant al coloration naturellement rosée de la peau et les charges abrasives ou exfoliantes..

### 1. Agents hydratants ou humectants

Comme agents humectants ou hydratants, on peut citer notamment le glycérol et ses dérivés, l'urée et ses dérivés notamment l'Hydrovance® commercialisée par National Starch, les acides lactiques, l'acide hyaluronique, les AHA, les BHA, le pidolate de sodium, le xylitol, la sérine, le lactate de sodium, l'ectoine et ses dérivés, le chitosane et ses dérivés, le collagène, le plancton, un extrait d'imperata cylindra commercialisé sous la dénomination Moist 24® par la société Sederma, des homopolymères d'acide acrylique comme le Lipidure-HM® de NOF corporation, le beta-glucan et en particulier le sodium carboxymethyl beta-glucane de Mibelle-AG-Biochemistry ; un mélange d'huiles de passiflore, d'abricot, maïs, et son de riz commercialisé par Nestlé sous la dénomination NutraLipids® ; un dérivé C-glycoside tel que ceux décrits dans la demande WO 02/051828 et en particulier le C-β-D-xylopyranoside-2-hydroxy-propane sous forme d'une solution à 30 % en poids en matière active dans un mélange eau/propylène glycol (60/40 % en poids) tel que le produit fabriqué par CHIMEX sous la dénomination commerciale « MEXORYL SBB® » ; une huile de rosier muscat commercialisée par Nestlé ; un extrait de micro-algue Prophyridium cruentum enrichi en zinc commercialisé par Vincience sous la dénomination Algualane Zinc®. ; des sphères de collagène et de chondroitine sulfate d'origine marine (Ateocollagen) commercialisées par la société Engelhard Lyon sous la dénomination sphères de comblement marines ; des sphères d'acide hyaluronique telles que celles commercialisées par la société Engelhard Lyon ; et l'arginine.

De préférence, on utilisera un agent hydratant choisi parmi l'urée et ses dérivés notamment l'Hydrovance® commercialisée par National Starch, l'acide hyaluronique, les AHA, les BHA, des homopolymères d'acide acrylique comme le Lipidure-HM® de NOF corporation, le beta-glucan et en particulier le sodium carboxymethyl beta-glucane de Mibelle-AG-Biochemistry ; un mélange d'huiles de passiflore, d'abricot, maïs, et son de riz commercialisé par Nestlé sous la dénomination NutraLipids® ; un dérivé de C-glycoside tel que ceux décrits dans la demande WO 02/051828 et en particulier le C-β-D-xylopyranoside-2-hydroxy-propane sous forme d'une solution à 30 % en poids en matière active dans un mélange eau/propylène glycol (60/40 % en poids) tel que le produit fabriqué par CHIMEX sous la dénomination commerciale « MEXORYL SBB® » ; une huile de rosier muscat commercialisée par Nestlé ; un extrait de micro-algue Prophyridium cruentum enrichi en zinc commercialisé par Vincience sous la dénomination Algualane Zinc®. ; des sphères de collagène et de chondroitine sulfate d'origine marine (Ateocollagen) commercialisées par la société Engelhard Lyon sous la dénomination sphères de comblement marines ; des sphères d'acide hyaluronique telles que celles commercialisées par la société Engelhard Lyon ; et l'arginine.

### 2. Agents desquamants

Par "agent desquamant", on entend tout composé capable d'agir :
- soit directement sur la desquamation en favorisant l'exfoliation, tel que les β-hydroxyacides (BHA), en particulier l'acide salicylique et ses dérivés (dont l'acide n-octanoyl 5-salicylique autrement nommé capryloyl salicylic acid en nom INCl) ; les α-hydroxyacides (AHA), tels que les acides glycolique, citrique, lactique, tartrique, malique ou mandélique ; l'acide 8-hexadécène-1,16-dicarboxylique ou acide 9-octadécène dioïque ; l'urée et ses dérivés; l'acide gentisique et ses dérivés ; les oligofucoses ; l'acide cinnamique ; l'extrait de Saphora japonica ; le resvératrol et certains dérivés d'acide jasmonique ;
   soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les composés aminosulfoniques et en particulier l'acide 4-(2-hydroxyethyl)piperazine-1-propanesulfonique (HEPES) ; l'acide 2-oxothiazolidine-4-carboxylique (procystéine) et ses dérivés ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

Comme autres agents desquamants utilisables dans la composition selon l'invention, on peut citer :
- les oligofructoses, l'EDTA et ses dérivés, les laminaria extract, le o-linoleyl-6D-glucose, l'acide (3-hydroxy-2pentylcyclopentyl) acétique, le trilactate de glycérol, l'O-octanyl-6'-D-maltose, la S carboxyméthyl cystéine, les dérivés siliciés de salicylate comme ceux décrits dans le brevet EP 0 796 861, les oligofucase comme ceux décrits dans le brevet EP 0 218 200, les sels d'acide 5-acyl salicylique, des actifs ayant des effets sur la transglutaminase comme dans le brevet EP 0 899 330,
- l'extrait de fleur de ficus opuntia indica comme l'Exfolactive® de Silab,
- l'acide 8-hexadécène 1,16-dicarboxylique,
- les esters de glucose et de vitamine F, et
- leurs mélanges.

Comme agents desquamants préférés, on pourra citer les beta-hydroxyacides, tel que l'acide n-octanoyl 5-salicylique ; l'urée ; les acides glycolique, citrique, lactique, tartrique, malique ou mandélique ; l'acide 4-(2-hydroxyethyl)piperazine-1-propanesulfonique (HEPES) ; l'extrait de Saphora japonica ; le miel ; le N-acétyl glucosamine ; le méthyl glycine diacétate de sodium, et leurs mélanges.

Encore plus préférentiellement on utilisera dans les compositions de l'invention un agent edsquamant choisi parmi l'acide n-octanoyl 5-salicylique ; l'urée ; l'acide 4-(2-hydroxyethyl)piperazine-1-propanesulfonique (HEPES) ; l'extrait de Saphora japonica ; le miel ; le N-acétyl glucosamine ; le méthyl glycine diacétate de sodium, et leurs mélanges.

### 3. Agents améliorant la fonction barrière

Comme agents améliorant la fonction barrière, on peut citer notamment l'arginine, la sérine, un extrait de Thermus thermophilus tel que le Vénucéane® de Sederma, un extrait de rhizome d'igname sauvage (dioscorea villosa) tel que l'Actigen Y® d'Active Organics, des extraits de plancton comme l'omega plancton® de Secma, des extraits de levure comme le Relipidium® de Coletica, un extrait de chataigne tel que la Recoverine® de Silab, un extrait de bourgeon de cèdre tel que le Gatuline Zen® de Gattefossé, des sphingosines comme la salicyloyl sphingosine vendue sous la dénomination « Phytosphingosine® SLC » par la société Degussa, un mélange de xylitol, de xylityl polyglycoside et de xylitan comme l'Aquaxyl® de Seppic, des extraits de solanacée comme le Lipidessence® de Coletica ; les huiles insaturées en oméga 3 telles que les huiles de rosier muscat et leurs mélanges.

On peut encore citer notamment les céramides ou dérivé, en particulier les céramides de type 2 (comme la N-oléoyldihydrosphingosine ), de type 3 (comme la stearoyl-4-hydroxysphinganine en nom INCl) et de type 5 (comme la N-2-hydroxypalmitoyldihydrosphingosine, ayant pour nom INCl :hydroxypalmytoyl sphinganine), les composés à base de sphingoïdes, les glycosphingolipides, les phospholipides, le cholestérol et ses dérivés, les phytostérols, les acides gras essentiels, le diacylglycérol, la 4-chromanone et dérivés de chromone la vaseline, la lanoline, les beures de karité, le cocoa butter, la lanoline, les sels PCA.

Comme agents préférés ayant un effet restructurant de la barrière cutanée, on citera un extrait de Thermus thermophilus, un extrait de rhizome d'igname sauvage (dioscorea villosa), un extrait de levure, un extrait de chataigne, un extrait de bourgeon de cèdre , l'arginine, la sérine, les céramides notamment de type 3 et 5 ; et leurs mélanges.

De préférence, on utilisera la sérine, l'arginine ou leur mélange.

### 4. Agents dépigmentants

Comme agents dépigmentants, on pourra citer notamment la vitamine C et ses dérivés et notamment la vit CG, CP et 3-O ethyl vitamine C, l'alpha et la béta arbutine, l'acide férulique, le lucinol et ses dérivés, l'acide kojique, le résorcinol et ses dérivés, l'acide tranexamique et ses dérivés, l'acide gentisique, l'homogentisate, le méthyl gentisate ou l'homogentisate , l'acide dioique, le D calcium panthéteine sulfonate, l'acide lipoique, l'acide ellagique, la vitamine B3, l'acide linoléique et ses dérivés, les céramides et leurs homologues, les dérivés de plantes comme la camomille, la busserole, la famille des aloe (vera, ferox, bardensis), de murier, de scutellaire ; une eau de fruit de kiwi (Actinidia chinensis) commercialisée par Gattefosse, un extrait de racine de Paeonia suffructicosa tel que celui commercialisé par la société lchimaru Pharcos sous la dénomination Botanpi Liquid B®, un extrait de sucre brun (Saccharum officinarum), tel que l'extrait de melasse commercialisé par la société Taiyo Kagaku sous la dénomination Molasses Liquid, sans que cette liste soit exhaustive.

Comme agents dépigmentants préférés, on utilisera la vitamine C et ses dérivés et notamment la vit CG, CP et 3-O ethyl vitamine C, l'alpha et la béta arbutine, l'acide férulique, l'acide kojique, le résorcinol et ses dérivés, le D calcium panthéteine sulfonate, l'acide lipoique, l'acide ellagique, la vitamine B3, une eau de fruit de kiwi (Actinidia chinensis) commercialisée par Gattefosse, un extrait de racine de Paeonia suffructicosa tel que celui commercialisé par la société lchimaru Pharcos sous la dénomination Botanpi Liquid B®.

### 5. Agents antioxydants

On peut citer notamment le tocophérol et ses esters, en particulier l'acétate de tocophérol ; l'acide ascorbique et ses dérivés, en particulier l'ascorbyl magnésium phosphate et l'ascorbyl glucoside ; l'acide férulique ; la sérine ; l'acide ellagique, la phlorétine, les polyphénols, les tanins, l'acide tannique, l'époigallocathéchines et les extraits naturels en contenant, les anthocyanes, les extraits de romarin, les extraits de feuilles d'olivier comme ceux de la société Silab, les extraits de thé vert, le resvératrol et ses dérivés, l'ergothinéine, la N acétylcystéine, un extrait d'algue brune pelvetia canaliculata comme la

Pelvetiane® de Secma, l'acide chlorogénique, la biotine, les chélatants, tels que le BHT, le BHA, le N,N'-bis(3,4,5-triméthoxybenzyl) éthylenediamine et ses sels ; l'idébénone, des extraits végétaux come le Pronalen Bioprotect TM de la société Provital ; le co enzyme Q10, les bioflavonoides, les SOD, le phytantriol, les lignanes, la mélatonine, les pidolates, le gluthation, le caprylyl glycol, la phloretine, le TotarolTM ou extrait de Podocarpus totara contenant du totarol (totara-8, 11, 13-trienol ou 2-phenanthrenol, 4b, 5, 6, 7, 8, 8a, 9, 10-octahydro-4b, 8, 8-trimethyl-1(1-methylethyl)- ; un extrait de jasmin tel que celui commercialisé par SILAB sous la dénomination Helisun® ; le laurate d'hesperitine tel que le Flavagrum PEG® de la société Engelhard Lyon ; un extrait de racine de Paeonia suffructicosa tel que celui commercialisé par la société Ichimaru Pharcos sous la dénomination Botanpi Liquid B® ; un extrait de litchi tel que l'extrait de péricarpe de litchi commercialisé par la société

Cognis sous la dénomination Litchiderm LS 9704®, un extrait de fruit de grenade (Punica Granatum), tel que celui commercialisé par la société Draco Natural products.

Comme autre agent anti-âge, on peut citer la DHEA et ses dérivés, l'acide boswellique, les extraits de romarin, les caroténoides (B carotène, zéaxanthine, lutéine), l'acide cystéique, les dérivés de cuivre, l'acide jasmonique

Comme agent antioxydant préféré, on utilisera notamment l'acide férulique ; la sérine ; la phlorétine, un extrait de grenade, la biotine, les chélatants, tels que le BHT, le BHA, le N,N'-bis(3,4,5-triméthoxybenzyl) éthylenediamine et ses sels , le caprylyl glycol, la phloretine, le TotarolTM, un extrait de jasmin tel que celui commercialisé par SILAB sous la dénomination Helisun® ; le laurate d'hesperitine tel que le Flavagrum PEG® de la société Engelhard Lyon ; un extrait de racine de Paeonia suffructicosa tel que celui commercialisé par la société lchimaru Pharcos sous la dénomination Botanpi Liquid B® .

### 6. Agents dermorelaxants ou dermodécontractants

On peut citer comme exemples le gluconate de manganèse et autres sels, l'adénosine, l'alvérine citrate et ses sels, la glycine, un extrait d'lris pallida, un hexapeptide (Argériline R de Lipotec) ou les sapogénines comme le Wild yam et les amines carbonylées décrites dans la demande EP1484052. Comme exemple de sapogénines on peut citer celles décrites dans la demande de brevet WO02/47650, en particulier le Wild yam, la diosgénine extrait notamment de Dioscorea opposita ou tout extrait refermant naturellement ou après traitement une ou plusieurs sapogénines (rhizome d'igname sauvage, feuille d'agave qui contient de l'hécogénine et la tigogénine, extrait de liliacées et plus particulièrement le Yacca ou le smilax contenant la smilagéine et la sarsapogénine, ou la racine de salsepareille) ou l' Actigen Y de la société Actives Organics ; ou le gingembre.
On peut également citer le DMAE (diméthyl MEA), les extraits de criste marine, de ciste de Montpellier, d'hélicryse, d'anis, de Para cress, un extrait d'Acmella Oleracea comme la Gatuline® expression de Gattefossé.

Comme agents dermorelaxants préférés, on citera l'adénosine, le gluconate de manganèse, le wild yam, la criste marine, la glycine et l'alvérine.

### 7. Agents anti-qlycation

Par « agent anti-glycation », on entend un composé prévenant et/ou diminuant la glycation des protéines de la peau, en particulier des protéines du derme, telles que le collagène.
Comme agents anti-glycation, on peut citer notamment les extraits végétaux de la famille des Ericaceae, tels qu'un extrait de myrtille (Vaccinium angusfifollium, Vaccinium myrtillus), par exemple celui vendu sous la dénomination « BLUEBERRY HERBASOL EXTRACT PG » par la société COSMETOCHEM, l'ergothionéine et ses dérivés, les hydroxystilbènes et leurs dérivés, tels que le resvératrol et le 3,3', 5,5'-tétrahydroxystilbène (ces agents anti-glycation sont décrits dans les demandes FR 2 802 425, FR 2 810 548, FR 2 796 278 et FR 2 802 420, respectivement), les dihydroxystilbènes et leurs dérivés, les polypeptides d'arginine et de lysine tels que celui vendu sous la dénomination « AMADORINE® » par la société SOLABIA, le chorhydrate de carcinine (commercialisé par Exsymol sous la dénomination « ALISTINO»), un extrait d'Hélianthus annuus comme l'Antiglyskin® de Silab, les extraits de vin tel que l'extrait de vin blanc en poudre sur support maltodextrine vendu sous la dénomination « Vin blanc déshydraté 2F » par la société Givaudan, l'acide thioctique (ou acide alpha lipoïque), un mélange d'extrait de busserole et de glycogène marin comme l'Aglycal LS 8777® de Laboratoires Sériobiologiques, un extrait de thé noir comme le Kombuchka® de Sederma et leurs mélanges.

Comme agents anti-glycation préférés, on citera les extraits de myrtille (Vaccinium myrtillus) et l'extrait de thé noir.

### 8. Agents stimulant la synthèse de macromolécules dermiques et/ou épidermiques et/ou empêchant leur dégradation :

Parmi les actifs stimulant les macromolécules du derme ou empêchant leur dégradation, on peut citer ceux qui agissent :
- soit sur la synthèse du collagène, tels que les extraits de Centella asiatica, les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ; les hormones végétales telles que les auxines et les lignanes ; l'acide folique ; et un extrait de Medicago sativa (alfafa) tel que celui commercialisé par SILBA sous la dénomination Vitanol® ; un extrait peptidique de noisette tel que celui commercialisé par la société Solabia sous la dénomination Nuteline C® ; et l'arginine.
   soit sur l'inhibition de la dégradation du collagène, en particulier des agents agissant sur l'inhibition des métalloprotéinases (MMP) telles que plus particulièrement les MMP 1, 2, 3, 9 . On peut citer : les rétinoïdes et dérivés, les extraits de medicago sativa tels que le Vitanol® de Silab, un extrait d'aphanizomenon flos-aquae (cyanophycée) commercialisée sous la dénomination Lanablue® par Atrium Biotechnologies, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon; un extrait de litchi tel que l'extrait de péricarpe de litchi commercialisé par la société Cognis sous la dénomination Litchiderm LS 9704®; la DIPALMITOYL HYDROXYPROLINE commercialisé par Seppic sous le nom SEPILIFT DPHP® : Baccharis genistelloide ou Baccharine commercialisé par SILAB, un extrait de moringa tel que l'Arganyl LS 9781® de Cognis ; l'extrait de sauge décrit dans la demande FR-A-2812544 de la famille des labiées (salvia officinalis de la société Flacksmann), l'extrait de Rhododendron, le blueberry extract, un extrait de vaccinium myrtillus tel que ceux décrits dans la demande FR-A-2814950.
- soit sur la synthèse de molécules appartenant à la famille des élastines (élastine et fibrilline), tels que : le retinol et dérivés en particulier le palmitate de rétinol ; l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie® ; un extrait peptidique de noisette tel que celui commercialisé par la société Solabia sous la dénomination Nuteline C®.
- soit sur l'inhibition de la dégradation de l'élastine tels que l'extrait peptidique de graines de Pisum sativum commercialisé par la société LSN sous la dénomination commerciale Parelastyl® ; les héparinoïdes ; et les composés N-acylaminoamides décrits dans la demande WO 01/94381 tels que l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valyl-glycine ou acetyl trifluoromethyl phenyl valylglycine, ou un ester de celui-ci avec un alcool en C1-C6. ; un extrait de peptides de riz tel que la Colhibin® de Pentapharm, ou un extrait de Phyllanthus emblica tel que l'Emblica® de Rona.
- soit sur la synthèse des glycosaminoglycannes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; l'extrait de Saccharomyces cerevisiae disponible notamment auprès de la société SILAB sous la dénomination commerciale Firmalift® ou auprès de la société LSN sous la dénomination commerciale Cytovitin®. ; un extrait de Laminaria ochroleuca tel que la Laminaïne® de Secma ; l'essence de Mamaku de Lucas Meyer, un extrait de cresson (Odraline® de Silab).
- soit sur la synthèse de la fibronectine, tels que l'extrait de zooplancton Salina commercialisé par la société SEPORGA sous la dénomination commerciale GP4G® ;
   l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil®.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® RC; et l'extrait de zooplancton Salina commercialisé par la société SEPORGA sous la dénomination commerciale GP4G® ; Tripeptide de Cuivre de PROCYTE ; ; un extrait peptidique de Voandzeia substerranea tel que celui commercialisé par la société Laboratoires Sérobiologiques sous la dénomination commerciale Filladyn LS 9397®.

De préférence on utilisera un actif stimulant la synthèse de macromolécules dermiques et/ou épidermiques et/ou empêchant leur dégradation choisi parmi les agents stimulant la synthèse des glycosaminoglycanes, les agents inhibant la dégradation de l'élastine, les agents stimulant la synthèse de la fibronectine, les agents stimulant la synthèse de macromolécules épidermiques, et leurs mélanges.

Encore plus préférentiellement, on utilisera un actif stimulant la synthèse des glycosaminoglycanes choisi parmi un extrait d'algue brune Padina pavonica, un extrait de Saccharomyces cerevisiae, un extrait de Laminaria ochroleuca, l'essence de Mamaku, un extrait de cresson et leurs mélanges.

Comme actifs préférés stimulant la synthèse de macromolécules dermiques et/ou épidermiques et/ou empêchant leur dégradation, on peut citer :
les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ;; l'acide folique ; un extrait de Medicago sativa (alfafa) tel que celui commercialisé par SILBA sous la dénomination Vitanol® ; un extrait peptidique de noisette tel que celui commercialisé par la société Solabia sous la dénomination Nuteline C® ; l'arginine ; . un extrait d'aphanizomenon flos-aquae (cyanophycée) commercialisée sous la dénomination Lanablue® par Atrium Biotechnologies, l'extrait de malt commercialisé par la société COLETICA sous la dénomination commerciale Collalift® le lycopène ; un extrait de litchi ; un extrait de moringa tel que l'Arganyl LS 9781® de Cognis ; un extrait de vaccinium myrtillus tel que ceux décrits dans la demande FR-A-2814950 ; : le retinol et dérivés en particulier le palmitate de rétinol ; l'extrait de Saccharomyces Cerivisiae commercialisé par la société LSN sous la dénomination commerciale Cytovitin® ; un extrait peptidique de noisette tel que celui commercialisé par la société Solabia sous la dénomination Nuteline C® ; l'acide {2-[acétyt-(3-triftuorométhylphényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valyl-glycine ou acetyl trifluoromethyl phenyl valylglycine, ou un ester de celui-ci avec un alcool en C1-C6. ; un extrait de peptides de riz tel que la Colhibin® de Pentapharm, ou un extrait de Phyllanthus emblica tel que l'Emblica® de Rona ; l'extrait d'algue brune Padina pavonica commercialisé par la société ALBAN MÜLLER sous la dénomination commerciale HSP3® ; l'extrait de Saccharomyces cerevisiae disponible notamment auprès de la société SILAB sous la dénomination commerciale Firmalift® ou auprès de la société LSN sous la dénomination commerciale Cytovitin®. ; un extrait de
Laminaria ochroleuca tel que la Laminaïne® de Secma ; l'essence de Mamaku de Lucas Meyer, l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® RC.

### 9. Agents stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; et les hormones végétales telles que les giberrellines et les cytokinines ; un extrait peptidique de noisette tel que celui commercialisé par la société Solabia sous la dénomination Nuteline C®.

De préférence on utilisera un agent favorisant la prolifération et/ou la différenciation des kératinocytes.

Les agents stimulant la prolifération des kératinocytes, utilisables dans la composition selon l'invention, comprennent notamment l'adénosine ; le phloroglucinol, l'extrait de feuille d'hydrangea macrophylla comme l'Amacha liquid E® d'Ichimaru Pharcos, un extrait de levure tel que le Stimoderm® de CLR ; l'extrait de Larrea divaricata tel que le Capislow® de Sederma, les mélanges d'extraits de papaye, de feuilles d'olivier et de citron tel que la Xyléine® de Vincience, l'extrait de feuille d'hydrangea macrophylla comme l'Amacha liquid E® d'lchimaru Pharcos, le rétinol et ses esters dont le palmitate de rétinyle, le phloroglucinol, les extraits de tourteaux de noix commercialisés par Gattefosse et les extraits de solanum tuberosum tel que le Dermolectine® commercialisé par Sederma.

Parmi les agents stimulant la différenciation des kératinocytes comprennent par exemple les minéraux tels que le calcium; la criste marine, un extrait peptidique de lupin tel que celui commercialisé par la société SILAB sous la dénomination commerciale Structurine®; le beta-sitosteryl sulfate de sodium tel que celui commercialisé par la société SEPORGA sous la dénomination commerciale Phytocohésine® ; et un extrait hydrosoluble de maïs tel que celui commercialisé par la société SOLABIA sous la dénomination commerciale Phytovityl® ; un extrait peptidique de Voandzeia substerranea tel que celui commercialisé par la société Laboratoires Sérobiologiques sous la dénomination commerciale Filladyn LS 9397® ; et les lignanes tels que le sécoisolaricirésinol, le rétinol et ses esters dont le palmitate de retinyl.

Comme agents stimulant la prolifération et/ou la différenciation des kératinocytes, on peut citer encore les oestrogènes tel que l'oestradiol et homologues ; les cytokines.

Comme actifs stimulant la prolifération des fibroblastes ou des kératinocytes et/ou la différenciation des kératinocytes préférés, on citera des protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; un extrait peptidique de noisette tel que celui commercialisé par la société Solabia sous la dénomination Nuteline C® ; l'adénosine ; le phloroglucinol, un extrait de levure tel que le Stimoderm® de CLR ; un extrait peptidique de lupin tel que celui commercialisé par la société SILAB sous la dénomination commerciale Structurine®; un extrait hydrosoluble de maïs tel que celui commercialisé par la société SOLABIA sous la dénomination commerciale Phytovityl® ; un extrait peptidique de Voandzeia substerranea tel que celui commercialisé par la société Laboratoires

Sérobiologiques sous la dénomination commerciale Filladyn LS 9397® ; le rétinol et ses esters dont le palmitate de retinyl.

### 10. Agents favorisant la maturation de l'enveloppe cornée

On pourra utiliser dans les compositions de l'invention des agents qui interviennent sur la maturation de l'enveloppe cornée qui s'altère avec l'âge et induit une diminution de l'activité des transglutaminases. On peut citer par exemple l'urée et ses dérivés et en particulier l'Hydrovance® de National Starch et les autres actifs mentionnés dans la demande L'OREAL FR2877220 (non publiée).

### 11. Inhibiteurs de NO-synthases

L'agent ayant une action d'inhibiteur de NO synthase peut être choisi parmi les OPC (oligomères procyanidoliques) ; les extraits de végétal de l'espèce Vitis vinifera notamment commercialisés par la société Euromed sous la dénomination Leucocyanidines de raisins extra, ou encore par la société Indena sous la dénomination Leucoselect®, ou enfin par la société Hansen sous la dénomination Extrait de marc de raisin ; les extraits de végétal de l'espèce Olea europaea de préférence obtenus à partir de feuilles d'olivier et notamment commercialisés par la société VINYALS sous forme d'extrait sec, ou par la société Biologia & Technologia sous la dénomination commerciale Eurol® BT ; les extraits d'un végétal de l'espèce Gingko biloba de préférence un extrait aqueux sec de ce végétal vendu par la société Beaufour sous le nom commercial Ginkgo biloba extrait standard et leurs mélanges.

### 12. Antagonistes des récepteurs périphériques des benzodiazépines (PBR)

On peut citer par exemple le 1-(2-chlorophenyl)-N-(1-methyl-propyl)-3-isoquinoline carboxamide ; les composés décrits dans les demandes WO03/030937, WO03/068753, dérivés de pyridazino[4, 5-b] indole-1-acétamide de formule générale (VII) tels que décrits dans le document WO00/44384.

### 13. Agents augmentant l'activité de la glande sébacée

On peut citer par exemple le dehydrojasmonate de méthyle, l'hécogénine, l'hédione, le le o-linoleyl-6D-glucose et leurs mélanges.

### 14. Agents stimulant le métabolisme énergétique des cellules

L'actif stimulant le métabolisme énergétique des cellules peut par exemple être choisi parmi la biotine, un extrait de Saccharomyces cerevisiae tel que le Phosphovital® de Sederma, le mélange de sels de sodium, de manganèse, de zinc et de magnésium d'acide pyrrolidone carboxylique comme le Physiogenyl® de Solabia, un mélange de gluconate de zinc, de cuivre et de magnésium tel que le Sepitonic M3® de Seppic et leurs mélanges ; un beta-glucan issu de Saccharomyces cerevisiae tel que celui commercialisé par la société Mibelle AG Biochemistry ;

### 15. Agents tenseurs

Par « agent tenseur » utilisable selon l'invention, on entend des composés susceptibles d'avoir un effet tenseur, c'est-à-dire pouvant tendre la peau.
De manière générale on entend par agent tenseur selon l'invention tous composés solubles ou dispersibles dans l'eau à une température allant de 25°C à 50°C à la concentration de 7% en poids dans l'eau ou à la concentration maximale à laquelle ils forment un milieu d'apparence homogène et produisant à cette concentration de 7% ou à cette concentration maximale dans l'eau une rétractation de plus de 15 % dans le test décrit ci-après.
La concentration maximale à laquelle ils forment un milieu d'apparence homogène est déterminée à ± 10% près et de préférence à ± 5% près.

On entend par 'milieu d'apparence homogène' un milieu ne présentant pas d'agrégats visibles à l'oeil nu.

Pour la détermination de ladite concentration maximale, l'agent tenseur est ajouté progressivement dans l'eau sous agitation à la défloculeuse à une température allant de 25°C à 50°C, puis le mélange est maintenu sous agitation pendant une heure. On observe ensuite après 24 heures si le mélange ainsi préparé est d'apparence homogène (absence d'agrégats visibles à l'oeil nu).

L'effet tenseur peut être caractérisé par un test in vitro de rétractation.
On prépare préalablement et tel que décrit précédemment un mélange homogène de l'agent tenseur dans l'eau, à la concentration de 7% en poids ou à la concentration maximale définie précédemment. 30µl du mélange homogène est déposé sur une éprouvette rectangulaire (10x40mm , donc présentant une largeur initiale L0 de 10 mm) d'élastomère ayant un module d'élasticité de 20 MPa et une épaisseur de 100µm. Après 3h de séchage à 22±3°C et 40±10% d'humidité relative HR, l'éprouvette d'élastomère présente une largeur rétractatée, notée L3h due à la tension exercée par l'agent tenseur déposé.

L'effet tenseur (ET) dudit agent est alors quantifié de la façon suivante :
ET' = (L0- L3h / L0)x100 en %
avec Lo = largeur initiale 10mm
et L3h = largeur après 3h de séchage

L'agent tenseur peut être choisi parmi :
les protéines végétales ou animales et leurs hydrolysats ;
les polysaccharides d'origine naturelle ;
les silicates mixtes ;
les particules colloïdales de charges inorganiques ;
les polymères synthétiques ;
et les mélanges de ceux-ci.

L'homme du métier saura choisir, dans les catégories chimiques listées ci-dessus, les matériaux répondant au test tenseur tel que décrit précédemment.

On peut citer notamment :
(a) les protéines et hydrolysats de protéines végétales, en particulier de maïs, de seigle, de froment, de sarrasin, de sésame, d'épautre, de pois, de fève, de lentille, de soja et de lupin,
(b) les polysaccharides d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,
(c) les silicates mixtes, notamment les phyllosilicates et en particulier les Laponites,
(d) les particules colloïdales de charge inorganique ayant un diamètre moyen en nombre compris entre 0,1 et 100 nm, de préférence entre 3 et 30 nm, et choisies par exemple parmi : la silice, les composites silice-alumine, l'oxyde de cérium, l'oxyde de zirconium, l'alumine, le carbonate de calcium, le sulfate de baryum, le sulfate de calcium, l'oxyde de zinc et le dioxyde de titane. Comme particules colloïdales composites silice-alumine utilisables dans les compositions selon l'invention, on peut citer par exemple celles commercialisées par la société Grace sous les noms de Ludox AM, Ludox AM-X 6021, Ludox HSA et Ludox TMA.
(e) les polymères synthétiques, tels que les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21,

L'agent tenseur sera présent dans la composition en une quantité efficace pour obtenir l'effet biologique recherché selon l'invention.

A titre d'exemple, l'agent tenseur peut être compris dans la composition selon l'invention en une teneur allant de 0,01 à 30% en poids de matière active, de préférence de 1 % à 30% en poids de matière active, par rapport au poids total de la composition.

Par « matière active », on entend exclure le milieu dans lequel l'agent tenseur se trouve éventuellement solubilisé ou en dispersion sous sa forme commerciale, par exemple dans le cas des dispersions de particules colloïdales.

On peut également utiliser, notamment pour complémenter et/ou potentialiser l'effet d'agents tenseurs, utiliser des agents augmentant l'expression des mécanorécepteurs, tels que des agents augmentant l'expression des intégrines. A titre d'exemple, on peut citer un extrait de graine de seigle, tel que celui commercialisé par SILAB sous la dénomination Coheliss©.

### 16. Agents liporestructurants

Par 'agents liporestrucurants', on entend selon l'invention des agents capables de stimuler la lipogénèse et favoriser la différenciation adipocytaire, permettant ainsi d'éviter ou de ralentir la fonte des graisses contenues dans les tissus de soutien de la peau, autrement nommée 'fonte de la lipo-structure de la peau'.
Par 'lipo-structure de la peau', on entend le réseau de cellules lipidiques qui forme les volumes sur lesquels la peau du visage repose et se moule.

Ces agents dont destinés à diminuer la perte de densité cutanée et/ou la fonte de la lipo-structure de la peau, en particulier au niveau des joues et du contour de l'oeil, et/ou éviter l'affaissement et/ou le creusement des volumes du visage, la perte de consistance de la peau et/ou son maintien, en particulier au niveau des joues et du contour de l'oeil, et/ou améliorer les volumes qui sous-tendent la peau du visage et/ou du cou, en particulier au niveau des joues, de l'ovale du visage et du contour de l'oeil, et/ou améliorer la densité, le rebondi et le maintien de la peau, en particulier au niveau des joues, de l'ovale du visage et du contour de l'oeil, et/ou refaçonner les traits du visage, en particulier l'ovale du visage.

Comme exemples d'agents liporestructurants, on peut citer notamment un extrait de thé noir, tel que l'extrait de thé noir fermenté commercialisé par Sederma sous al dénomination Kombuchka®, et un extrait d'Artemisia abrotanum, tel que celui commercialisé par Sllab sous la dénomination Pulpactyl®.

### 17. Agents amincissants

Comme agents amincissants (lipolytiques), on peut citer notamment la caféine, théophylline et ses dérivés, la théobromine, la séricosine, l'acide asiatique, l'acéfylline, l'aminophylline, le chloroéthylthéophylline, le diprofylline, le diniprophylline, l'étamiphylline et ses dérivés, l'étofylline, la proxyphylline ; les extraits de thé, de café, de guarana, de maté, de cola (Cola Nitida) et notamment l'extrait sec de fruit de guarana (Paulina sorbilis) contenant 8 à 10 % de caféine ; les extraits de lierre grimpant (Hedera Helix), d'arnica (Arnica Montana L), de romarin (Rosmarinus officinalis N), de souci (Calendula officinalis), de sauge (Salvia officinalis L), de ginseng (Panax ginseng), de millepertuis (Byperycum Perforatum), de fragon (Ruscus aculeatus L), d'ulmaire (Filipendula ulmaria L), d'orthosiphon (Orthosiphon Stamincus Benth), de bouleau (Betula alba), de cécropia et d'arganier ; les extraits de ginkgo biloba, les extraits de prêle, les extraits d'escine, les extraits de cangzhu, les extraits de chrysanthellum indicum, les extraits de dioscorés riches en diosgénine ou la diosgénine ou hécogénine pure et leurs dérivés, les extraits de Ballote, les extraits de Guioa, de Davallia, de Terminalia, de Barringtonia, de Trema, d'Antirobia, l'extrait de petit grain de bigarrade ; un extrait de coques de fèves de cacao (theobroma cacao) tel que celui commercialisé par Solabia sous la dénomination Caobromine®.

### 18. Agents favorisant la microcirculation cutanée

L'actif agissant sur la microcirculation cutanée peut être utilisé pour éviter le ternissement du teint et/ou améliorer l'aspect du contour de l'oeil, en particulier diminuer les cernes. Il peut être choisi par exemple parmi un extrait d'écorce de pin maritime comme le Pycnogénol® de chez Biolandes, le gluconate de manganèse (Givobio GMn® de Seppic), un extrait d'Ammi visnaga comme la Visnadine d'lndena, l'extrait de lupin (Eclaline® de Silab), le couplage protéine de blé hydrolysée/acide palmitique avec acide palmitique comme l'Epaline 100 des Laboratoires carilène, l'extrait de fleur de bigarade (Remoduline® de Silab), la vitamine P et ses dérivés comme le methyl-4 esculétol mono-éthanoate de sodium vendu sous la dénomination Permethol® par la société Sephytal, les extraits de ruscus, de marron d'inde, de lierre, de ginseng et de mélilot, la caféine, le nicotinate et ses dérivés, la lysine et ses dérivés comme l'Asparlyne® de Solabia, un extrait de thé noir tel que le Kombuchka de Sederma ; les sels de rutine : un extrait d'algue de corallina officinalis tel que celui commercialisé par CODIF ; et leurs mélanges.

Comme agents préférés favorisant la microcirculation cutanée, on citera la caféine, un extrait de fleur de bigarade, un extrait de thé noir, les sels de rutine, un extrait d'algue de corallina officinalis.

### 19. Agents apaisants ou anti-irritants

On entend par agent apaisant un composé permettant de réduire la sensation de picotements, de démangeaisons ou de tiraillements de la peau.

Comme agents apaisants utilisables dans la composition selon l'invention, on peut citer : les oligomères procyannidoliques, les vitamines E, C B5, B3, la caféine et ses dérivés, les triterpènes pentacycliques et les extraits de plantes les contenant, l'acide b-glycyrrhétinique et ses sels ou dérivés (le stearyl glycyrrhetate, l'acide 3-stéaroyloxy glycyrrhétique, l'acide glycyrrhétinique monoglucuronide) ainsi que les plantes en contenant (ex : Glycyrrhiza glabra), l'acide oléanolique et ses sels, l'acide ursolique et ses sels, l'acide boswellique et ses sels, l'acide bétulinique et ses sels, un extrait de Paeonia suffruticosa et/ou lactiflora, un extrait de Laminaria saccharina, les extraits de Centella asiatica, l'huile de Canola, le bisabolol, le diesterphosphorique de vitamine E et C comme le Sepivital EPC® de Seppic, les extraits de camomille, l'allantoïne, les huiles insaturées en oméga 3 telles que les huiles de rosier muscat, de cassis, d'Ecchium, de poisson, l'huile de calophilum, des extraits de plancton, la capryloyl glycine, un mélange d'extrait de fleur de nénuphar et de palmitoylproline tel que celui vendu sous la dénomination "Seppicalm VG®" par la société Seppic, un extrait de Boswellia serrata, un extrait de Centipeda cunnighami tel que celui vendu sous la dénomination "Cehami PF®" par la société TRI-K Industries, un extrait de graines de tournesol en particulier l'Hélioxine® de Silab, un extrait de graines de Linum usitatissimum comme la Sensiline® de Silab, les tocotriénols, le piperonal, un extrait d'Epilobium angustifolium tel que celui vendu sous la dénomination "Canadian Willowherb Extract" par la société FYTOKEM PRODUCTS, l'aloe vera, les phytostérols, l'eau de bleuet, l'eau de rose, un extrait de menthe, en particulier de feuilles de menthe comme le Calmiskin® de Silab, les dérivés d'anis, les bactéries filamenteuse comme Vitreoscilla filiformis tel que décrit dans le brevet EP 761 204 et commercialisé par Chimex sous la dénomination Mexoryl SBG®, un extrait de pétales de rose comme le Rose Flower Herbasol® extract de la société Cosmetochem, le beurre de karité, un mélange de fraction de cire de graine d'orge obtenue par CO2 supercritique, de beurre de karité et d'huile d'argan comme le "Stimu-tex AS®" de Pentapharm, les sels alcalino-terreux notamment le strontium, un extrait fermenté d'Alteromonas commercialisé sous la dénomination ABYSSINE® par la société Atrium Biotechnologies ; les eaux thermales du bassin de Vichy, telles que les eaux provenant des sources Célestins, Chomel, Grande-Grille, Hôpital, Lucas et Parc, et de préférence l'eau de la source Lucas ; un extrait d'écorce d'Eperua falcata tel que celui commercialisé par la société COGNIS sous al dénomination Eperuline® ; un extrait de racine de Paeonia suffructicosa tel que celui commercialisé par la société Ichimaru Pharcos sous la dénomination Botanpi Liquid B® ; et leurs mélanges.

Comme agents apaisants préférés selon l'invention, on utilisera :
l'acide b-glycyrrhétinique et ses sels ou dérivés (le stearyl glycyrrhetate, l'acide 3-stéaroyloxy glycyrrhétique, l'acide glycyrrhétinique monoglucuronide) ainsi que les plantes en contenant (ex : Glycyrrhiza glabra) ; l'acide ursolique et ses sels ; les extraits de Centella asiatica, l'huile de Canola, le bisabolol ; les extraits de camomille, l'allantoïne , un mélange d'extrait de fleur de nénuphar et de palmitoylproline tel que celui vendu sous la dénomination "Seppicalm VG®" par la société Seppic ; l'aloe vera, l'eau de rose, un extrait de menthe, en particulier de feuilles de menthe comme le Calmiskin® de Silab, les bactéries filamenteuse comme Vitreoscilla filiformis tel que décrit dans le brevet EP 761 204 et commercialisé par Chimex sous la dénomination Mexoryl SBG®, un extrait de pétales de rose comme le Rose Flower Herbasol® extract de la société Cosmetochem, le beurre de karité, un extrait fermenté d'Alteromonas commercialisé sous la dénomination ABYSSINE® par la société Atrium Biotechnologies ; les eaux thermales du bassin de Vichy, telles que les eaux provenant des sources Célestins, Chomel, Grande-Grille, Hôpital, Lucas et Parc, et de préférence l'eau de la source Lucas ; un extrait d'écorce d'Eperua falcata tel que celui commercialisé par la société COGNIS sous al dénomination Eperuline® ; un extrait de racine de Paeonia suffructicosa tel que celui commercialisé par la société Ichimaru Pharcos sous la dénomination Botanpi Liquid B® ; et leurs mélanges.

### 20. Agents sébo-réqulateurs ou anti-séborrhéiques

Par agents "séborégulateurs ou anti-séborrhéiques ", on entend notamment des agents capables de réguler l'activité des glandes sébacées.

On peut citer notamment :
- l'acide rétinoïque, le peroxyde de benzoyle, le soufre, la vitamine B6 (ou pyridoxine ), le chlorure de sélénium, la criste marine ;
- les mélanges d'extrait de canelle, de thé et d'octanoylglycine tel que le Sepicontrol A5 TEA® de chez Seppic ;
- le mélange de canelle, de sarcosine et d'octanoylglycine, commercialisé notamment par la société SEPPIC sous la dénomination commerciale Sepicontrol A5® ;
- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ;
- les dérivés de cuivre et en particulier le pidolate de cuivre tel que Cuivridone® de Solabia ;
- des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
- les extraits de reine des prés (spiraea ulamaria) tel que celui vendu sous la dénomination Sébonormine® par la société Silab ;
- les extraits d'algue laminaria saccharina tel que celui vendu sous la dénomination Phlorogine® par la société Biotechmarine ;
- les mélanges d'extraits de racines de pimprenelle (sanguisorba officinalis/poterium officinale), de rhizomes de gingembre (zingiber officinalis) et d'écorce de cannelier (cinnamomum cassia) tel que celui vendu sous la dénomination Sebustop® par la société Solabia ;
- les extraits de graines de lin tel que celui vendu sous la dénomination Linumine® par la société Lucas Meyer ;
- les extraits de Phellodendron tels que ceux vendu sous la dénomination Phellodendron extract BG par la société Maruzen ou Oubaku liquid B par la société Ichimaru Pharcos ;
- les mélanges d'huile d'argan, d'extrait de serenoa serrulata (saw palmetto) et d'extrait de graines de sésame tel que celui vendu sous la dénomination Regu SEB® par la société Pentapharm ;
- les mélanges d'extraits d'epilobe, de terminalia chebula, de capucine et de zinc biodisponible (microalgues) tel que celui vendu sous la denomination Seborilys® par la société green tech ;
- les extraits de Pygeum afrianum tel que celui vendu sous la dénomination Pygeum afrianum sterolic lipid extract par la société Euromed ;
- les extraits de serenoa serrulata tels que ceux vendus sous les dénomination Viapure Sabal par la société Actives International, ou ceux vendus par la société Euromed ;
- les mélanges d'extraits de plantain, de berberis aquifolium et de salicylate de sodium tels que celui vendu sous la dénomination Seboclear® par la société Rahn ;
- l'extrait de clou de girofle tel que celui vendu sous la dénomination Clove extract Powder par la société Maruzen ;
- l'huile d'argan telle que celle vendue sous la dénomination Lipofructyl® par les Laboratoires Sérobiologiques ;
- les filtrats de protéine lactique tels que celui vendu sous la dénomination Normaseb® par la société Sederma ;
- les extraits d'algue laminaria, tel que celui vendu sous la dénomination Laminarghane® par la société Biotechmarine ;
- les oligosaccharides d'algue laminaria digitata tel que celui vendu sous la dénomination Phycosaccharide AC par la société Codif ;
- les extraits de sucre de canne tel que celui commercialisé sous la dénomination Policosanol® par la société Sabinsa ;
- l'huile de schiste sulfonée, telle que celle vendue sous la dénomination Ichtyol Pale® par la société Ichthyol ,
- les extraits d'ulmaire (spiraea ulmaria) tels que celui vendu sous la dénomination Cytobiol® Ulmaire par la société Libiol ;
- l'acide sébacique, notamment vendu sous la forme d'un gel de polyacrylate de sodium sous la dénomination Sebosoft® par la société Sederma ;
- les glucomannanes extraits de tubercule de konjac et modifié par des chaînes alkylsulfonates tel que celui vendu sous la dénomination Biopol Beta par la société Arch Chemical ;
- les extraits de Sophora angustifolia, tels que ceux vendus sous la dénomination Sophora powder ou Sophora extract par la société Bioland ;
- les extraits de cinchona succirubra bark tel que celui vendu sous la dénomination le Red bark HS par la société Alban Muller ;
- les extraits de quillaja saponaria tel que celui vendu sous la dénomination Panama wood HS par la société Alban Muller ;
- la glycine greffée sur chaîne undécylénique, telle que celle vendue sous la dénomination Lipacide UG OR par la société Seppic ;
- le mélange d'acide oléanolique et d'acide nordihydroguaïarétique, tel que celui vendus sous la forme d'un gel sous la dénomination AC.Net par la société Sederma ;
- l'acide phthalimidoperoxyhexanoïque ;
- le citrate de trialkyle(C12-C13) vendu sous la dénomination COSMACOL® ECI par la société Sasol ; le citrate de trialkyle(C14-C15) vendu sous la dénomination COSMACOL® ECL par la société Sasol ;
- l'acide 10-hydroxydécanoïque, et notamment les mélanges d'acide 10-hydroxydécanoïque, d'acide sébacique et de 1,10-décandiol tels que celui vendu sous la dénomination Acnacidol® BG par la société Vincience ; et
- leurs mélanges.

Comme actif anti-séborrhéique préférés, on peut citer :
- le peroxyde de benzoyle, la vitamine B6 (ou pyridoxine ),
- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ;
- les extraits de reine des prés (spiraea ulamaria) tel que celui vendu sous la dénomination Sébonormine® par la société Silab;
- les extraits d'algue laminaria saccharina tel que celui vendu sous la dénomination Phlorogine® par la société Biotechmarine;
- les mélanges d'extraits de racines de pimprenelle (sanguisorba officinalis/poterium officinale), de rhizomes de gingembre (zingiber officinalis) et d'écorce de cannelier (cinnamomum cassia) tel que celui vendu sous la dénomination Sebustop® par la société Solabia;
- l'extrait de clou de girofle tel que celui vendu sous la dénomination Clove extract Powder par la société Maruzen ;
- les filtrats de protéine lactique tels que celui vendu sous la dénomination Normaseb® par la société Sederma ;
- les extraits d'ulmaire (spiraea ulmaria) tels que celui vendu sous la dénomination Cytobiol® Ulmaire par la société Libiol;
- l'acide sébacique, notamment vendu sous la forme d'un gel de polyacrylate de sodium sous la dénomination Sebosoft® par la société Sederma ;
- la glycine greffée sur chaîne undécylénique, telle que celle vendue sous la dénomination Lipacide UG OR par la société Seppic;
- le citrate de trialkyle(C12-C13) vendu sous la dénomination COSMACOL® ECI par la société Sasol; le citrate de trialkyle(C14-C15) vendu sous la dénomination COSMACOL® ECL par la société Sasol;
- l'acide 10-hydroxydécanoïque, et notamment les mélanges d'acide 10-hydroxydécanoïque, d'acide sébacique et de 1,10-décandiol tels que celui vendu sous la dénomination Acnacidol® BG par la société Vincience;
- et leurs mélanges.

Préférentiellement, l'actif anti-séborrhéique est choisi parmi:
- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc; et de préférence le pyrrolidone carboxylate de zinc (ou pidolate de zinc) ou le salicylate de zinc ;
- l'extrait de clou de girofle tel que celui vendu sous la dénomination Clove extract Powder par la société Maruzen ;
- la glycine greffée sur chaîne undécylénique, telle que celle vendue sous la dénomination Lipacide UG OR par la société Seppic;
- le citrate de trialkyle(C12-C13) vendu sous la dénomination COSMACOL® ECI par la société Sasol ; le citrate de trialkyle(C14-C15) vendu sous la dénomination COSMACOL® ECL par la société Sasol;
- et leurs mélanges.

L'actif anti-séborrhéique est par exemple présent dans une teneur allant de 0,1 à 10 % en poids, de préférence de 0,1 à 5 % en poids, et préférentiellement de 0,5 % à 3 % en poids, par rapport au poids total de la composition.

### 21. Agents astringents

Par "agents astringents", on entend selon l'invention des agents permettant de lutter contre la dilatation des follicules sébacés.

Comme agents astringents utilisables dans la composition selon l'invention, on peut citer des extraits de pulpe de champignon (polyporus officinalis) comme le "Laricyl LS8865® " de Cognis, des extraits de Terminalia catappa et sambucus nigra comme le Phytofirm LS9120® de Cognis, des extraits de noix de galle comme le Tanlex VE® de Ichimaru Pharcos, l'hydroxychlorure d'aluminium, les extraits de centella (ex Plantactiv centella de Cognis), le chlorure de dicétyl diméthyl ammonium comme le Varisoft 432 CG® de Degussa, les extraits de marron d'inde, les extraits de mauve, les extraits d'Hammamelis, des extraits d'amandes douces, de racines de guimauve et de graines de lin comme l'Almondermin LS 3380® de Cognis, les extraits de bardane, les extraits d'ortie, les extraits de bouleau, les extraits de prêle, les extraits de camomille comme ceux vendus sous la dénomination Extrapone 9 special® par la société Symrise, les extraits de scutellaria, les extrais d'Ulmaire (par exemple le Cytobiol Ulmaire de Libiol), un mélange d'extraits de gingembre blanc, de prêle, d'ortie, de romarin, de yucca comme l'Herb extract B1348® de Bell flavors & fragrances, les extraits d'acacia, d'orme, de saule blanc, de canelle, de bouleau, de reine des prés, les sapogénines de panama, le phenolsulfonate de zinc de Interchemical, des extraits de gentiane, de concombre, de noyer, le mélange d'extraits de Ratanhia, de pamplemousse, de grindelia et de galle de chêne comme l'Epilami® de Alban Muller.

Comme agents astringents préférés selon l'invention, on utilisera les extraits de scutellaria, les extraits d'ulmaire, les extraits de reine des près, les extraits de gentiane, les extraits de bardane et leurs mélanges.

### 22. Agents cicatrisants

Comme exemples d'agents cicatrisants, on peut citer notamment:
l'allantoine, l'urée, certains acides aminés comme l'hydroxyproline, l'arginine, la sérine, et aussi des extraits de lys blanc (comme le Phytélène Lys 37EG 16295 de Indena), un extrait de levures comme le cicatrisant LS LO/7225B des Laboratoires Sériobiologiques), l'huile de tamanu, l'extrait de saccharomyces cerevisiae comme le Biodynes® TRF® de Arch Chemical, les extraits d'avoine, le chitosane et dérivés comme le glutamate de chitosane, les extraits de carotte, l'extrait d'artemia comme le GP4G® de Vincience, l'acexamate de sodium, des extraits de lavandin, des extraits de propolis, l'acide ximeninique et ses sels, l'huile de rosa rugosa, des extraits de souci comme le Souci Ami® Liposolible d'Alban Muller, des extraits de prêle, les extraits d'écorce de citron comme l'Herbasol® citron de Cosmetochem, des extraits d'helichryse, des extraits de millefeuilles, et l'acide folique.

Comme agents cicatrisants préférés selon l'invention, on utilisera l'arginine, la sérine, l'acide folique, l'huile de tamanu, l'acexamate de sodium, des extraits de prêle, des extraits d'helichryse, et leurs mélanges.

### 23. Agents anti-inflammatoires

Comme agents anti-inflammatoires particuliers utilisables selon l'invention, on peut citer la cortisone, l'hydrocortisone, l'indométhacine, la bétaméthasone, l'acide azéalique, l'acétominophène, le diclofénac, le propionate de clobetasol, l'acide folique; un extrait d'écorce d'Eperua falcata tel que celui commercialisé par la société COGNIS sous al dénomination Eperuline®; un extrait de racine de Paeonia suffructicosa tel que celui commercialisé par la société Ichimaru Pharcos sous la dénomination Botanpi Liquid B®; et leurs mélanges.

Comme agent anti-inflammatoire préféré, on citera l'acide azélaique, l'acide folique, un extrait d'écorce d'Eperua falcata tel que celui commercialisé par la société COGNIS sous al dénomination Eperuline® ; un extrait de racine de Paeonia suffructicosa tel que celui commercialisé par la société Ichimaru Pharcos sous la dénomination Botanpi Liquid B®; et leurs mélanges.

### 24. Agents anti-acné

Dans un aspect avantageux de l'invention, la composition peut comporter en outre au moins un actif anti-acné.
Par "actif anti-acné", on entend notamment tout actif ayant des effets sur la flore spécifique des peaux grasses tels que par exemple le Propionibacterium acnes (P acnes).

Ces effets peuvent être bactéricides.
A titre d'actifs antibactéricide, on peut citer notamment:
- les actifs et conservateurs à activité anti-microbienne cités dans la demande DE10324567, incorporée dans la présente invention par référence,
- l'acide asiatique,
- le sel de monoéthanolamine du 1-hydroxy-4-methyl 6-trimethylpentyl 2-pyridone (nom INCI: piroctone olamine), notamment vendu sous la dénomination Octopirox® par la société Clariant ;
- l'acide citronellique, l'acide périllique (ou acide 4-isopropenylcyclohex-1-ènecarboxylique),
- le 2-éthyl hexyle éther de glycérol (nom INCl: ethylhexylglycerine), par exemple vendu sous la dénomination Sensiva SC 50® par la société Shulke & Mayr,
- le caprylate/caprate de glyceryle, par exemple vendu sous la dénomination Capmul MCM® par la société ABITEC;
- le phosphosilicate de calcium et de sodium, notamment vendu sous les dénominations Bioactive glasspowder® et Actysse Premier BG® par la société Schott Glass ;
- les particules à base d'argent, par exemple celle vendues sous la dénomination Métashine ME 2025 PS® par la société Nippon Sheet Glass ;
- l'extrait de cône de houblon (Humulus Lupulus) obtenu par extraction CO2 supercritique tel que celui vendu sous la dénomination HOP CO2-TO extract® par la société Flavex Naturextrakte,
- l'extrait de Millepertius obtenu par extraction CO2 supercritique tel que celui vendu sous la dénomination ST John's Wort CO2-TO extract® par la société Flavex Naturextrakte,
- le mélange d'extraits de racines de scutellaria baicalensis, de paeonia suffruticosa et de glycyrrhiza glabra, tel que celui vendu sous la dénomination BMB- CF® par la société Naturogin,
- l'extrait d'arganier comme l'Argapure LS9710® de chez COGNIS;
- les extraits de feuilles de busserole comme celui vendu sous la dénomination "Melfade-J" par la société Pentapharm ;
- l'acide 10-hydroxy-2 décanoique tel que l'Acnacidol P® de chez Vincience, l'ursolate de sodium, l'acide azélaique, le di-iodo-méthyl p-tolylsulfone tel que l'Amical Flowable® de chez Angus, la poudre de malachite, l'oxyde de zinc tel que Zincare® de chez Elementis GMBH, l'acide octadécènedioïque tel que l'Arlatone dioic DCA® de chez Uniqema; l'acide ellagique; le 2,4,4'-trichloro-2'-hydroxy diphényl éther (ou triclosan), la 1-(3',4'-dichlorophényl)-3-(4'-chlorophényl)urée (ou triclocarban), le 3,4,4'-trichlorocarbanilide, le 3',4',5'-trichlorosalicylanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopirox, le ciclopiroxolamine, l'acide undécylenique et ses sels, le peroxyde de benzoyle, l'acide 3-hydroxy benzoïque, l'acide 4-hydroxy benzoïque, l'acide phytique, la N-acétyl-L-cystéine, l'acide lipoïque, l'acide azélaïque et ses sels, l'acide arachidonique, le résorcinol, le 3,4,4'-trichlorocarbanalide, l'octoxyglycérine ou octoglycérine, l'octanoylglycine tel que Lipacid C8G® de Seppic, le caprylyl glycol, l'acide 10-hydroxy-2-décanoïque, le dichlorophenyl imidazol dioxolan et ses dérivés décrits dans la demande de brevet WO9318743, l'iodopropynyl butylcarbamate, le 3,7,11-triméthyldodéca-2,5,10-triénol ou farnesol, les phytosphingosines; les sels d'ammonium quaternaires comme les sels de cetyltrimethylammonium, les sels de cétylpyridinium, et
- leurs mélanges.

On peut également citer certains tensioactifs ayant un effet antimicrobien comme le cocoampho acétate de sodium ou diacetate disodium tel que le Miranol C2M CONC NP, les bétaines comme la cocoyl bétaine Genagen KB de Clariant, le lauryl ether sulfate de sodium comme l'Emal 270 D de Kao, le décyl glucoside comme le Plantacare 2000 UP, les malate de dialcools C12-13 ramifiés comme le Cosmacol EMI, les monoesters de propylène glycol comme monolaurate, monocaprylate, monocaprate de propylène glycol, le lauryl dimethylamine betaine comme le Empigen BB/LS ainsi que les polyammonium quaternaires comme le Quaternium-24 ou Bardac 2050 de Lonza et ceux décrits dans le brevet FR 0 108 283 et leurs mélanges.

Comme agents antimicrobiens préférés, on utilisera dans les compositions de l'invention un agent choisi parmi l'octoglycérine ou octoxyglycérine, l'acide 10-hydroxy-2-décanoïque, et leurs mélanges.

D'autres actifs anti-acné additionnels peuvent être ajoutés aux actifs anti-acnés précités.

On peut notamment citer les actifs présentant des effets anti-adhésion bactérienne ou bien agissant sur le biofilm des bactéries pour éviter leur multiplication.

Comme agents prévenant et/ou réduisant l'adhésion des micro-organismes, on peut citer notamment : le phytanetriol et ses dérivés tels que décrits dans la demande de brevet EP 1 529 523, les huiles végétales telles de l'huile de germe de blé, l'huile de calendula, l'huile de ricin, l'huile d'olive, l'huile d'avocat, l'huile d'amande douce, l'huile d'arachide, l'huile de jojoba, l'huile de sésame, l'huile d'amande d'abricot, l'huile de tournesol, l'huile de macadamia, décrites dans le brevet EP 1 133979, ou encore certains tensioactifs tels que le cocoamphodiacétate disodique, le cocoate de glycéryle oxyéthyléné (7 OE), l'hexadécénylsuccinate 18, le palmitate d'octoxyglycéryle, le béhénate d'octoxyglycéryle, l'adipate de dioctyle, le PPG-15 stéaryl éther, le tartrate de dialcools C12-C13 ramifiés décrits dans le brevet EP 1 129 694 et leurs mélanges.
En particulier vis-à-vis de la propagation du P acnes, ou en tant qu'actifs agissant sur le biofilm des bactéries pour éviter leur prolifération, on peut citer le pentylène glycol, le nylon-66 (fibres de polyamides 66), l'huile de son de riz , l'alcool polyvinylique tel que Celvol 540 PV alcohol® de chez Celanese Chemical, l'huile de colza telle que Akorex L® de chez Karlshamns, et les dérivés de fructose et leurs mélanges.

L'actif agissant contre l'acné peut être présent dans une teneur allant de 0,01 à 10 % en poids, de préférence de 0,05 à 5 % en poids par rapport au poids total de la composition.

En fonction de la nature et/ou de la solubilité des actifs précités, l'homme du métier saura choisir le mode de réalisation le plus adapté selon l'invention. Comme actifs lipophiles utilisables dans le kit ou l'une au moins des compositions de l'invention, on peut citer notamment le D α tocophérol, le DL α tocophérol, l'acétate de D α tocophérol, l'acétate DL αtocophérol, le palmitate d'ascorbyle, les glycérides de vitamine F, les vitamines D, la vitamine D2, la vitamine D3, rétinol, les esters de rétinol, le palmitate de rétinol, le propionate de rétinol, les carotènes dont le β carotène, le D panthénol, le farnesol, l'acétate de farnesyle, l'acide salicylique et ses dérivés comme l'acide n-octanoyl-5 salicylique, les alkylesters d'α hydroxyacides tels que l'acide citrique, l'acide lactique, l'acide glycolique, l'acide asiatique, l'acide madécassique, l'asiaticoside, l'extrait total de centella asiatica, l'acide β glycyrrhétinique, l'α bisabolol, les céramides comme le 2 oleoylamino-1,3 octadécane, le phytanetriol, les phospholipides d'origine marine riches en acides gras essentiels polyinsaturés, l'éthoxyquine, l'extrait de romarin, l'extrait de mélisse, le quercetine, l'extrait de microalgues séchées, l'huile essentielle de bergamotte, l'octylmethoxycinnamate, le butylméthoxydibenzoylméthane, l'octyl triazone, le di tertiobutyl-3,5 hydroxy-4 benzylidène 3 camphre, les antibiotiques, les antifongiques, les anesthésiques, les analgésiques, les antiseptiques, les antiviraux, les pesticides, les herbicides, et leurs mélanges.

Les actifs cosmétiques et/ou dermatologiques seront présents dans le kit ou l'une des compositions selon l'invention en une teneur allant de 0,001 % à 20% en poids par rapport au poids total de la composition, de préférence de 0,01 % à 10%, encore plus préférentiellement de 0,5 à 5% et de préférence encore de 0,1 à 1 % en poids par rapport au poids total de la composition.

Pour des applications 'peeling', les teneurs en actifs cosmétiques et/ou dermatologiquies pourront aller de 1 à 50% en poids par rapport au poids total de la composition, de préférence de 1 à 30% en poids par rapport au poids total de la composition.

Les peelings sont un moyen bien connu pour améliorer l'aspect et/ou la texture de la peau et/ou du cuir chevelu, notamment améliorer l'éclat et l'homogénéité du teint et/ou diminuer les irrégularités visibles et/ou tactiles de la peau, et en particulier pour améliorer l'aspect de surface de la peau, pour atténuer les lentigo actiniques, les marques d'acné ou de varicelle, ainsi que pour prévenir, atténuer ou lutter contres les signes du vieillissement cutané, et notamment pour lisser les irrégularités de la texture de la peau, telles les rides et les ridules.

Ils ont pour effet d'enlever une partie superficielle de la peau à traiter (épiderme et éventuellement couche superficielle du derme), par des méthodes chimiques.

### AUTRES INGREDIENTS ADDITIONNELS

Pour complémenter et/ou optimiser les effets conférés par les actifs cosmétiques et/ou dermatologiques cités ci-dessus sur les matières kératiniques, il peut être avantageux d'intégrer dans les compositions de l'invention d'autres ingrédients additionnels.

En particulier, ces ingrédients addditionnels pourront conférer un effet immédiat visuel qui sera relayé par l'effet biologique des actifs cités ci-dessus. Ils pourront également, via une action mécanique (ex : charges abrasvies), amplifier l'effet des actifs biologiques cités ci-dessus.

Ainsi la composition selon l'invention pourra comprendre en outre au moins un agent choisi parmi des agents matifiants, des charges à effet flouteur, des agents fluorescents, des agents favorisant la coloration naturellement rosée de la peau, des charges abrasives ou agents exfoliants, et leurs mélanges.

### Agents matifiants

Par "agent matifiant", on entend des agents destinés à rendre la peau visiblement plus mate, moins brillante.

L'effet matifiant de l'agent et/ou de la composition le contenant peut notamment être évalué à l'aide d'un gonioréflectomètre, en mesurant le rapport R entre la réflexion spéculaire et la réflexion diffuse. Une valeur de R inférieure ou égale à 2 traduit généralement un effet matifiant.

L'agent matifiant pourra notamment être choisi parmi un amidon de riz ou un amidon de maïs, la kaolinite, le talc, un extrait de graines de potiron, des microbilles de cellulose, des fibres végétales, des fibres synthétiques, en particulier de polyamides, des microsphères de copolymères acryliques expansées, des poudres de polyamides, les poudres de silice, les poudres de polytétrafluoroéthylène, les poudres de résine de silicone, les poudres de polymères acryliques, les poudres de cire, les poudres de polyéthylène, les poudres d'organopolysiloxane réticulé élastomère enrobées de résine de silicone, les poudres composites de talc/dioxyde de titane/alumine/silice, les poudres de silicates mixtes amorphes, les particules de silicate et notamment de silicate mixte, et leurs mélanges.

Comme exemples d'agents matifiants, on peut citer notamment:
- l'amidon de riz ou de maïs, en particulier un aluminium starch octenyl succinate commercialisé sous la dénomination Dry Flo® par la société National Starch,
- la kaolinite ;
- les silices ;
- le talc ;
- un extrait de graines de potiron tel que commercialisé sous la dénomination Curbilene® par la société Indena;
- des microbilles de cellulose telles que décrites dans la demande de brevet EP 1 562 562 ;
- des fibres, telles que des fibres de soie, de coton, de laine, de lin, de cellulose extraites notamment du bois, des légumes ou des algues, de polyamide (Nylon®), de cellulose modifiée, de poly-p-phénylène téréphtamide, en acrylique, de polyoléfine, de verre, de silice, d'aramide, de carbone, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges décrites dans la demande de brevet EP 1 151 742 ;
- des microsphères de copolymères acryliques expansées telles que celles commercialisées par la société EXPANCEL sous les dénominations EXPANCEL 551®,
- des charges à effet optique telles que décrites dans la demande de brevet FR 2 869 796, en particulier :

- les poudres de polyamides (Nylon®), comme par exemple les particules de Nylon 12 du type Orgasol d'Arkema de taille moyenne 10 microns et d'indice de réfraction 1,54,
- les poudres de silice, comme par exemple les Silica beads SB150 de Miyoshi de taille moyenne 5 microns et d'indice de réfraction 1,45,
- les poudres de polytétrafluoroéthylène, comme les PTFE ceridust 9205F de Clariant de taille moyenne 8 microns et d'indice de réfraction 1,36,
- les poudres de résine de silicone comme les Silicon resin Tospearl 145A de GE Silicone de taille moyenne 4,5 microns et d'indice de réfraction 1,41,
- les poudres de copolymères acryliques, notamment de poly(méth)acrylate de méthyle, comme les particules PMMA Jurymer MBI de Nihon Junyoki de taille moyenne 8 microns et d'indice de réfraction 1,49, ou les particules Micropearl M100® et F 80 ED® de la société Matsumoto Yushi-Seiyaku,
- les poudres de cire comme les particules Paraffin wax microease 114S de micropowders de taille moyenne 7 microns et d'indice de réfraction 1,54,
- les poudres de polyéthylène, notamment comprenant au moins un copolymère éthylène/acide acrylique, et en particulier constituées de copolymères éthylène/acide acrylique comme les particules Flobeads EA 209 de Sumitomo (de taille moyenne 10 microns et d'indice de réfraction 1,48),
- les poudres d'organopolysiloxane réticulé élastomère enrobées de résine de silicone, notamment de résine silsesquioxane, comme décrit par exemple dans le brevet US 5 538 793. De telles poudres d'élastomère sont vendues sous les dénominations "KSP-100", "KSP-101", "KSP-102", "KSP-103", "KSP-104", "KSP-105" par la société SHIN ETSU, et
- les poudres composites de talc/dioxyde de titane/alumine/silice comme celles vendues sous la dénomination Coverleaf® AR-80 par la société Catalyst & chemicals,
- leurs mélanges,
- des composés absorbant et/ou adsorbant le sébum tels que décrits dans la demande de brevet FR 2 869 796. On peut citer notamment :

- les poudres de silice, comme par exemple les microsphères de silice poreuses vendues sous la dénomination "SILICA BEADS SB-700" commercialisées par la société MYOSHI, les "SUNSPHERE® H51", "SUNSPHERE® H33" , "SUNSPHERE® H53" commercialisées par la société ASAHI GLASS ; les microsphères de silice amorphe enrobées de polydiméthylsiloxane vendues sous la dénomination "SA SUNSPHERE® H-33" et "SA SUNSPHERE® H-53" commercialisées par la société ASAHI GLASS ;
- les poudres de silicates mixtes amorphes, notamment d'aluminium et de magnésium, comme par exemple celle commercialisée sous la dénomination "NEUSILIN UFL2" par la société Sumitomo.
- les poudres de polyamides (nylon®), comme par exemple "l'ORGASOL® 4000" commercialisé par la société Arkema, et
- les poudres de polymères acryliques, notamment de polyméthacrylate de méthyle, comme par exemple le "COVABEAD® LH85" commercialisé par la société WACKHERR ; de polyméthacrylate de méthyle/diméthacrylate d'éthylène glycol, comme par exemple le "DOW CORNING 5640 MICROSPONGE® SKIN OIL ADSORBER" commercialisé par la société DOW CORNING, ou le "GANZPEARL® GMP-0820" commercialisé par la société GANZ CHEMICAL ; de polyméthacrylate d'allyle/diméthacrylate d'éthylène glycol, comme par exemple le "POLY-PORE® L200" ou le "POLY-PORE® E200" commercialisés par la société AMCOL ; de copolymère diméthacrylate d'éthylène glycol/méthacrylate de lauryle, comme par exemple le "POLYTRAP® 6603" commercialisé de la société DOW CORNING;
- les particules de silicate, telle que la silicate d'alumine ;
- les particules de silicates mixtes, telles que :

- les particules de silicate d'aluminium et de magnésium, telles que la saponite ou silicate de magnésium et d'aluminium hydraté avec un sulfate de sodium commercialisée sous la dénomination commerciale Sumecton® par la société Kunimine;
- le complexe silicate de magnésium, hydroxyéthylcellulose, huile de cumin noir, huile de courge et phospholipides ou Matipure® de Lucas Meyer, et
- leurs mélanges.

Comme agents matifiants préférés, on pourra utiliser selon l'invention un extrait de graines de potiron, un amidon de riz ou de mais, la kaolinite, des silices, le talc, les poudres de polyamides, les poudres de polyethylènes, les poudres de copolymères acryliques, les microsphères de copolymères acryliques expansées, les microbilles de résines de silicones, les particules de silicate mixte et leurs mélanges.

### Charges à effet flouteur

Ces charges peuvent être tout matériau susceptible de modifier les rides par ses propriétés physiques intrinsèques et de les masquer. Ces charges peuvent notamment modifier les rides par un effet tenseur, un effet de camouflage, ou un effet de floutage.

En tant que charge, on peut donner à titre d'exemples les composés suivants:
- les microparticules poreuses de silice comme par exemple les Silica Beads® SB 150 et SB 700 de Myochi de taille moyenne de 5µm et les SUNSPHERES® série H d'Asahi Glass comme les H33, H51 de taille respectivement de 3,5 et 5 µm.
- les particules hémisphériques creuses de résines de silicones comme les NLK 500®, NLK 506® et NLK 510® de Takemoto Oil and Fat, notamment décrites dans EP-A-1579849,
- les poudres de résine de silicone comme par exemple les SILICON Resin Tospearl® 145 A DE GE silicone de taille moyenne de 4,5µm.
- les poudres de copolymères acryliques, notamment de poly(meth)acrylate de méthyle comme par exemple les particules PMMA Jurimer MBI® de Nihon Junyoki de taille moyenne de 8µm, les sphères creuses de PMMA vendues sous la dénomination COVABEAD® LH 85 par la société Wackherr et les microsphères de vinylidène/acrylonitrile/méthacrylates de méthylène expansées vendues sous la dénomination Expancel®.
- les poudres de cires comme les particules Paraffin wax microloase® 114S de Micropowders de taille moyenne de 7µm.
- les poudres de polyéthylènes notamment comprenant au moins un copolymère éthylène/acide acrylique comme par exemple les FLOBEADS® EA 209 E de Sumimoto de taille moyenne de 10µm.
- les poudres d'organopolysiloxanes élastomériques réticulées enrobées de résine de silicone notamment de silsesquioxane sous la dénomination KSP 100®, KSP 101®, KSP 102®, KSP 103®, KSP 104® et KSP 105® par la société Shin Etsu.
- les poudres composites de talc/dioxyde ou de titane/alumine/silice comme par exemple les Coverleaf AR 80® de la société Catalyst & Chemical.
- le talc, le mica, le kaolin, la lauryl glycine, les poudres d'amidon réticulés par l'anhydride octéanyl succinate, le nitrure de bore, les poudres de polytétrafluoroéthylène, le carbonate de calcium précipité, le carbonate de l'hydrocarbonate de magnésium, le sulfate de baryum, l'hydroxyapatite, le silicate de calcium, le dioxyde de cérium et les microcapsules de verre ou de céramiques.
- les fibres hydrophiles ou hydrophobes synthétiques ou naturelles, minérales ou organiques telles que des fibres de soie, de coton, de laine, de lin, de cellulose extraites notamment du bois, des légumes ou des algues, de polyamide (Nylon®), de cellulose modifiée, de poly-p-phénylène téréphtamide, en acrylique, de polyoléfine, de verre, de silice, d'aramide, de carbone, de polytétrafluoroéthylène (Téflon®), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges décrites dans la demande de brevet EP 1 151 742.
- les silicones réticulés élastomères sphériques comme comme les Trefil E-505C® ou E-506 C® de chez Dow Corning.
- les charges abrasives qui par effet mécanique apportent un lissage du microrelief cutané, telles que la silice abrasive comme par exemple Abrasif SP® de Semanez ou des poudres de noix ou de coques (abricot, noix par exemple de Cosmétochem).

Les charges ayant un effet sur les signes du vieillissement sont notamment choisies parmi des microparticules poreuse de silice, des particules hémisphériques creuses de silicones, des poudres de résine de silicone, des poudres de copolymères acryliques, des poudres de polyéthylènes, des poudres d'organopolysiloxanes élastomériques réticulées enrobées de résine de silicone, des poudres composites de talc/dioxyde de titane/alumine/silice, le carbonate de calcium précipité, le carbonate de l'hydrocarbonate de magnésium, le sulfate de baryum, l'hydroxyapatite, le silicate de calcium, le dioxyde de cérium et les microcapsules de verre ou de céramiques, les fibres de soie, de coton, et leurs mélanges.

La charge peut être une charge « soft focus ».
Par charge « soft-focus », on entend une charge qui en plus donne de la transparence au teint et un effet flou. De préférence, les charges « soft-focus » ont une taille moyenne des particules inférieure ou égale à 15 microns. Ces particules peuvent être de toutes formes et en particulier être sphériques ou non sphériques. De préférence encore, ces charges sont non sphériques. Les charges « soft-focus » peuvent être choisies parmi les poudres de silice et silicates, notamment d'alumine, les poudres de type polyméthyl méthacrylate (PMMA), le talc, les composites silice/TiO2 ou silice/oxyde de zinc, les poudres de polyéthylène, les poudres d'amidon, les poudres de polyamides, les poudres de copolymères styrène/acrylique, les élastomères de silicone, et leurs mélanges.
En particulier, on peut citer le talc de taille moyenne en nombre inférieure ou égale à 3 microns, par exemple du talc de taille moyenne en nombre de 1,8 micron et notamment celui vendu sous la dénomination commerciale Talc P3® par la société Nippon Talc, la poudre de Nylon® 12, notamment celle vendue sous le dénomination Orgasol 2002 Extra D Nat Cos® par la société Atochem, les particules de silice traitées en surface par une cire minérale 1 à 2 % (nom INCI: hydrated silica (and) paraffin) telles que celles commercialisées par la société Degussa, les microsphères de silice amorphe, telles que celles vendues sous la dénomination Sunsphère par exemple de référence H-53® par la société Asahi Glass, et les micro-billes de silice telles que celles vendues sous la dénomination SB-700® ou SB-150® par la société Miyoshi, cette liste n'étant pas limitative.
La concentration de ces charges ayant un effet sur les signes du vieillissement dans les compositions selon l'invention peut être comprise entre 0,1 et 40 %, voire entre 0,1 et 20 % en poids par rapport au poids total de la composition.

### Agents fluorescents

On entend par agent fluorescent une substance qui, sous l'effet de rayons ultraviolet et/ou de la lumière visible, ré-émet dans le visible la portion de lumière qu'elle a absorbé sous la même couleur que celle qu'elle reflète naturellement. La couleur réfléchie naturellement est ainsi renforcée par la couleur ré émise et apparaît extrêmement brillante.

On peut citer par exemple les résines colorées de polyamide et/ou de formaldéhyde/benzoguanamine et/ou de melamine/formaldéhyde/sulfonamide, parmi les co-condensats aminotriazine/formaldéhyde/sulfonamide colorés et/ou parmi les paillettes polyester métallisées et/ou leurs mélanges. Ces pigments fluorescents peuvent aussi se présenter sous la forme de dispersions aqueuses de pigments fluorescents.

On peut encore citer le co-condensat aminotriazine/formaldehyde/sulfonamide fluorescent coloré en rose de taille moyenne des particules 3-4 microns vendu sous la dénomination commerciale « Fiesta Astral Pink FEX-1 » et le co-condensat aminotriazine/formaldehyde/sulfonamide fluorescent coloré en bleu de taille moyenne des particules 3-4,5 microns vendu sous la dénomination commerciale « Fiesta Comet Blue FTX-60 » par la société Swada ou encore la résine benzoguanamine/formaldéhyde recouverte de résine formaldéhyde/urée et colorée en jaune vendue sous la dénomination commerciale « FB-205 Yellow » et la résine benzoguanamine/formaldéhyde recouverte de résine formaldéhyde/urée et colorée en rouge vendue sous la dénomination commerciale « FB-400 Orange Red » par la société UK SEUNG CHEMICAL, la résine polyamide colorée en orange vendue sous la dénomination commerciale « Flare 911 Orange 4 » par la société Sterling Industrial Colors.

Les substances fluorescentes sont de préférence présentes dans la composition, à une teneur allant de 0,1 à 20%, de préférence de 0,1 à 15%, de préférence encore de 0,5 à 3% en poids, par rapport au poids total de la composition.
Lorsque les substances fluorescentes organiques sont blanches, on les appelle également des azurants optiques.

L'azurant optique a pour effet d'intensifier l'éclat et aviver les teintes des compositions cosmétiques les comprenant à l'application sur la peau.
Parmi les azurants optiques, on peut plus particulièrement citer les dérivés du stilbène, en particulier les polystyrylstilbènes et les triazinstilbènes, les dérivés coumariniques, en particulier les hydroxycoumarines et les aminocoumarines, les dérivés oxazole, benzoxazole, imidazole, triazole, pyrazoline, les dérivés du pyrène et les dérivés de porphyrine et/ou leurs mélanges.

De tels composés sont par exemple disponibles sous les dénominations commerciales Tinopal SOP et Uvitex OB auprès de la société CIBA GEIGY.

Les azurants optiques préférentiellement utilisés sont le 4,4'-bis[(4,6-dianilino-1,3,5-triazin-2-yl)amino]stilbène-2,2'-disulfonate de sodium, le 2,5 thiophène di-yl bis(5 ter-butyl-1,3 benzoxazole), le di-styryl-4,4' biphényle sulfonate disodique et/ou leurs mélanges.

### Agents favorisant la coloration naturellement rosée de la peau

On peut citer notamment :
- un agent autobronzant, c'est-à-dire un agent qui, appliqué sur la peau, notamment sur le visage, permet d'obtenir un effet de bronzage d'apparence plus ou moins semblable à celui qui peut résulter d'une exposition prolongée au soleil (bronzage naturel) ou sous une lampe UV ;
- un agent de coloration additionnel, c'est-à-dire tout composé ayant une affinité particulière pour la peau lui permettant de conférer à cette dernière une coloration durable, non-couvrante (à savoir n'ayant pas tendance à opacifier la peau) et qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un pigment de maquillage; et leurs mélanges.

Comme exemples d'agents autobronzants, on peut citer notamment:
la dihydoxyacétone (DHA),
l'érythrulose, et
l'association d'un système catalytique formé de:
sels et oxydes de manganèse et/ou de zinc, et hydrogénocarbonates alcalins et/ou alcalinoterreux.

Les agents autobronzants sont généralement choisis parmi les composés mono ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342. On utilisera de préférence la DHA.

La DHA peut être utilisée sous forme libre et/ou encapsulée par exemple dans des vésicules lipidiques telle que des liposomes, notamment décrits dans la demande WO 97/25970.

D'une manière générale, l'autobronzant est présent en une quantité allant de 0,01 à 20 % en poids, et de préférence en quantité comprise entre 0,1 et 10 % du poids total de la composition.

On peut encore utiliser d'autres colorants qui permettent de modifier la couleur produite par l'agent autobronzant.

Ces colorants peuvent être choisis parmi les colorants directs synthétiques ou naturels.

Ces colorants peuvent être choisis par exemple parmi les colorants rouges ou oranges du type fluorane tels que ceux décrits dans la demande de brevet FR2840806. On peut citer par exemple les colorants suivants :
- le tetrabromofluoroscéine ou éosine connue sous le nom CTFA : CI 45380 ou Red 21
- la phloxine B connue sous le nom CTFA : CI 45410 ou Red 27
- la diiodofluorescéine connue sous le nom CTFA CI 45425 ou Orange 10;
- la dibromofluorescéine connue sous le nom CTFA : CI 45370 ou Orange 5.
- le sel de sodium de la tetrabromofluoroscéine connue sous le nom CTFA : CI 45380 (Na salt) ou Red 22
- le sel de sodium de la phloxine B connu sous le nom CTFA : CI 45410 (Na salt) ou Red 28
- le sel de sodium de la diiodofluorescéine connu sous le nom CTFA : CI 45425 (Na salt) ou Orange 11;
- l'érythrosine connu sous le nom CTFA : CI 45430 ou Acid Red 51.
- la phloxine connu sous le nom CTFA : CI 45405 ou Acid Red 98.
   Ces colorants peuvent être également choisis parmi les antraquinones , le caramel, le carmin, le noir de charbon, les bleus azulènes, le methoxalène, le trioxalène, le guajazulène, le chamuzulène, le rose de bengale, la cosine 10B, la cyanosine, la daphinine.
   Ces colorants peuvent être également choisis parmi les dérivés indoliques comme les monohydroxyindoles tels que décrits dans le brevet FR2651126 ( ie : 4-, 5-, 6- ou 7-hydroxyindole) ou les di-hydroxyindoles tels que décrits dans le brevet EP-B-0425324 (ie : 5,6-dihydroxyindole, 2-méthyl 5,6-dihydroxyindole, 3-méthyl 5,6-dihydroxyindole, 2,3-diméthyl 5,6-dihydroxyindole);

### Charges abrasives ou agents exfoliants

Comme agents exfoliants utilisables dans des compositions rincées selon l'invention, on peut citer par exemple des particules exfoliantes ou gommantes d'origine minérale, végétale ou organique. Ainsi, on peut utiliser par exemple des billes ou de la poudre de polyéthylène, de la poudre de nylon, de la poudre de polychlorure de vinyle, de la pierre ponce, des broyats de noyaux d'abricots ou de coques de noix, de la sciure de bois, des billes de verre, l'alumine, et leurs mélanges. On peut citer aussi l'Exfogreen® de Solabia (extrait de bambou), des extraits d'akenes de fraises (Akenes de fraise de Greentech), de la poudre de noyau de pêche, la poudre de noyau d'abricot, et enfin dans le domaine des poudres végétales à effet abrasif, citons la poudre de noyaux d'airelles (cranberry).

Comme charges abrasives ou agents exfoliants préférés selon l'invention, on citera la poudre de noyaux de pêche, la poudre de noyaux d'abricot, la poudre de noyaux d'airelles, les extraits d'akènes de fraise, les extraits de bambou.

Les exemples qui suivent servent à illustrer l'invention. Dans ces exemples, les quantités des ingrédients compositions sont données en % pondéral par rapport au poids total de la composition.

### Exemples de synthèse:

### Exemple 1 : synthèse de l'acide 4-methyl-1,2-dithiolane-4-carboxyligue (composé 1)

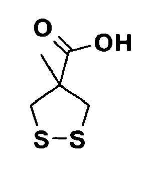

8g d'acide dichloropivalique sont placés dans un tricol de 250ml surmonté d'un réfrigérant et d'une ampoule de coulée. L'acide est dissout dans 80ml d'eau, en ajoutant lentement 4,6g de Na₂CO₃. Une solution de 10,7 g de thioacétate de potassium est additionnée goutte à goutte, le milieu réactionnel est porté au reflux. 14,9 g de Na₂CO₃ sont ajoutés et le milieu chauffé de nouveau au reflux. Après disparition du produit de départ, 7,3 ml de DMSO sont ajoutés avant de chauffer au reflux. Le dithiolane est obtenu après acidification par précipitation et séchage sous vide du solide. On obtient un solide jaune pâle RMN 1 H (400 MHz, DMSO-d6): δ ppm 3,69 (d, 2H), 2,95 (d, 2H), 1,53 (s, 3H),
ESI-:
[(M, H) -] = 163 m/z

### Exemple 2: Synthèse de l'octyl 4-methyl-1,2-dithiolane-4-carboxylate (composé 9)

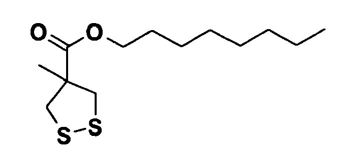

Dans un tricol de 100 ml, sont chargés sous argon 1g d'acide (24) puis 0,8 ml du 1-chloro-N,N-2-triméthyl-propenylamine (27) à l'aide d'une seringue dans 20 ml de dichlorométhane. le mélange est agité pendant 1h puis introduit goutte à goutte par une ampoule de coulée dans un milieu réactionnel à -5°C contenant 1.28ml de triéthylamine, 0,96 ml d'octanol et 20 ml de dichlorométhane. On laisse agiter. Le milieu réactionnel est ensuite lavé à l'eau (3 X30 ml). La phase aqueuse est extraite avec 3X10ml d'AcOEt . Les phases organiques réunies sont lavées avec 30ml de solution aqueuse saturée de NaCI puis séchées sur Na₂SO₄, filtrées puis concentrées sous vide (500mbar, T=40°C) au rotavapor. Le brut obtenu est une huile jaune (m=1,25g). La purification se fait par chromatographie flash sur une colonne de silice (mSiO2=40g- Eluant par gradient Heptane/AcOEt : 100/0 puis 98/2.)

Après concentration des fractions au rotavapor (P=100 mbar, T=40°C), on obtient 1,08 g d'attendu pur. Huile jaune Rendement =66% ; Rf (ester)= 0,16 (Eluant : Cyclohexane);
RMN 1 H (400 MHz, DMSO-d6): δ ppm 4,08 (t, 2H), 3,57 (d, 2H), 3,02 (d, 2H), 1,58 (m, 2H), 1,40 (s, 3H), 1.29 (m, 10H), 0,86 (t, 3H) MS m/z (M+, 277; M+23, 299).

Les manipulations suivantes ont été faites dans les mêmes conditions précédemment décrites, seul le nucléophile varie.

### Exemple 3: Synthèse du S-[2-(acetylamino)ethyl] 4-methyl-1,2-dithiolane-4-carbothioate (Composé 13) (hors invention)

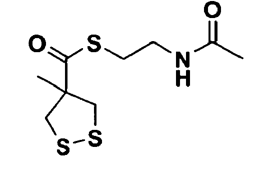

Méthode identique à l'exemple 2: le nucléophile utilisé est la N-acétylcystéamine (0,64ml).

La purification se fait par chromatographie flash sur une colonne de silice (mSiO2=40g- Eluant par gradient linéaire DCM/MeOH : 100/0 puis 98/2.) Après concentration des fractions au rotavapor (P=200 mbar, T=40°C), on obtient 0,32 g d'un mélange de l'attendu avec le N,N-2-triméthyl-propionamide (28).

Liquide jaune épaisse Rendement=10%; Rf (attendu)= 0,3; Eluant: DCM/MeOH : 95/5 ; RMN 1 H (DMSO-d6): δ ppm 8,03 (t, NH), 3,57 (d, 2H), 3,18 (d.t 2H), 3,10 (d, 2H), 2,.96 (m, 2H), 1,79 (s, 3H), 1,43 (s, 3H) ; MS m/z (M+, 266;M+23, 288).

### Exemple 4: Synthèse du N-(2-hydroxyethyl)-4-methyl-1,2-dithiolane-4-carboxamide (Composé 14)

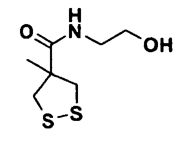

Méthode identique à l'exemple 2 : le nucléophile utilisé est l'éthanolamine (0,36ml). Après filtration du milieu réactionnel, on obtient un brut, huile jaune (m=1,850g).

La purification se fait par chromatographie flash sur une colonne de silice (Eluant par gradient linéaire DCM/MeOH: 100/0 puis 98/2. Après concentration des fractions au rotavapor (P=500 mbar, T=40°C), on obtient 800 mg d'attendu pur, huile jaune R=65%
Rf (attendu)= 0,43 ; Eluant : DCM/MeOH : 9/1 ;
RMN 1 H (DMSO-d6): δ ppm 7,80 (t, NH), 4,64 (t, OH), 3,53 (d. 2H), 3,40 (d.t, 2H), 3,14 (m, 2H), 2,99 (d, 2H), 1,34 (s, 3H) ; MS m/z (M+, 208 ; M+23, 230).

### Exemple 5 : Synthèse du 4-methyl-1,2-dithiolane-4-carboxamide (Composé 2)

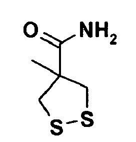

Méthode (ex5-a) identique à l'exemple 2 : le nucléophile utilisé est l'ammoniaque dans l'isopropanol (9,5ml). Après filtration du milieu réactionnel, on obtient un brut, huile jaune (m=1.853g). La purification se fait par chromatographie flash sur une colonne de silice (Eluant : DCM). Après concentration des fractions au rotavapor (P=600 mbar, T=40°C), on obtient 500 mg d'attendu pur, solide jaune Rendement =52%.

Alternativement, méthode (ex5-b), à une solution de 1g de composé 1 dans le THF avec 1,2 équivalent de triéthylamine, sont ajoutés à 0°C 1,2 équivalent de chloroformiate d'isobutyle. Après 2 heures à température ambiante, le milieu réactionnel est ajouté à de l'ammoniac en solution refroidie, soit dans l'eau à 28% soit 2N dans l'isopropanol. Le milieu est agité à température ambiante le temps nécessaire avant concentration sous vide. Le brut est alors repris dans le toluène pour conduire par précipitation au composé 2. Rendement = 60%
Rf (attendu)= 0,45 ; Eluant : DCM/MeOH : 95/5 ; RMN 1 H (DMSO-d6): δ ppm 7,38 (s, NH), 7,13 (s, NH), 3,53 (d. 2H), 2,97 (d, 2H), 1,34 (s, 3H) ; ESI- : [(M, H) -] = 162 m/z ; ESI+ : [(M, Na) +] = 186 m/z ; ESI+ : [(M, H) +] = 164 m/z ; ESI+ : [(M, Na, MeOH) +] =218 m/z

### Exemple 6 : Synthèse du N-(2,3-dihydroxypropyl)-4-methyl-1,2-dithiolane-4-carboxamide (Composé 15)

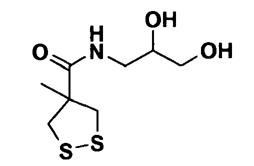

Méthode identique à l'exemple 2 : la quantité d'acide de départ engagé est 0,.25 g et le nucléophile utilisé est le diméthyldioxalaneméthanamine (0.2ml). On obtient 110mg d'attendu pur, huile jaune Rendement=26% Rf (attendu)= 0,51 ; Eluant : DCM/MeOH : 95/5 ; RMN 1 H (DMSO-d6): δ ppm 7.77 (t, 1H :NH), 3,55 (d.d 4H, H3 :diastéréoisomères), 3,5 (m, 4H : H7 diastéréoisomères), 3,20 (m, 2H : H8 : diastéréoisomères), 3,05 (d.d, 2H:H9 et H9'), 2,99 (d.d, 4H, H5), 1,35 (s, 12H, H10+H11: diastéréoisomères), 0,9 (d, 3H,
H6); MS m/z (M+23, 300).

70mg du produit pur protégé sous forme d'acétonide et environ 5 g de résine Dowex sont engagés dans une solution de 3ml d'eau et 2ml de THF. Le mélange réactionnel est agité à TA pendant 20h, puis à 40°C pendant 40h.

Le milieu réactionnel avec de la résine est filtré sous vide et lavé avec 3*10 ml d'eau puis 2X10ml d'EtOH. Le filtrat est ensuite concentré au rotavapor (P=200 mbar, T=40°C), on obtient 30 mg d'huile jaune présentant 2 diastéréoisomères. Rf (attendu)= 0,24 ; Eluant: DCM/MeOH : 9/1 ; RMN 1H (DMSO-d6): δ ppm 7,80 (t, 1 H : NH), 4.73 (d, OH), 4.50 (t, OH), 3,55 (d. 4H), 3,4 (m. 2H), 3.,2 (m, 1 H), 3.1 (m, 2H), 2,99 (d, 4H), 1,35 (s, 3H) ; MS m/z (M+, 208 ; M+23, 230).

### Exemple 7: Synthèse du N-heptyl-4-methyl-1,2-dithiolane-4-carboxamide (Composé 10)

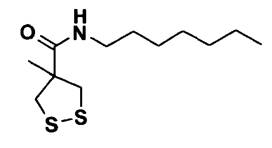

Méthode identique à l'exemple 2 : le nucléophile est 0.22ml de n-Heptylamine.
Le brut obtenu est une huile jaunâtre (m=0,27g). La purification se fait par chromatographie flash sur une colonne de silice (mSiO2=12g-Eluant DCM/MeOH : 99/1).

Après concentration des fractions au rotavapor (P=500 mbar, T=40°C), on obtient 0.21 g d'attendu pur, huile jaune Rendement=54%. Rf (attendu)= 0.5 ; Eluant : DCM/MeOH : 99/1 ; RMN 1 H (DMSO-d6): δ ppm 7.78 (t, NH), 3,53 (d. 2H), 3.1 (d.t, 2H), 2,97 (d, 2H), 1,41 (t.t. 2H), 1,34 (s, 3H), 1,23 (m, 8H), 0,85 (t, 3H) ; MS m/z (M+, 262 ; M+23, 284)

### Exemple 8: Synthèse du Methyl 2-H(4-methyl-1,2-dithiolan-4-yl)carbonyl]amino}-4-(methylsulfanyl)butanoate (Composé 12)

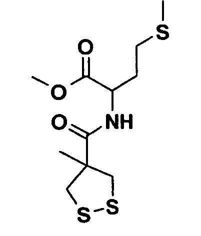

Méthode identique à l'exemple 2 : le nucléophile est la L-méthionine methyl-ester.

RMN 1 H (DMSO-d6): δ ppm 8,13 (d, NH), 4,4 (m. 1 H), 3.63 (s, 3H), 3,58 (m, 2H), 3,02 (m, 2H), 2,5 (m, 2H), 2.04 (s, 3H), 1,96 (m, 2H), 1,38 (s, 3H); MS m/z (M+, 310; M+23, 332)

### Exemple 9: Synthèse du N-butyl-4-methyl-1,2-dithiolane-4-carboxamide (Composé 11)

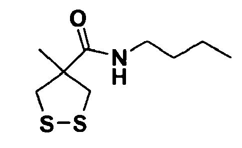

A 1g d'acide 1,2 dithiolane 4-méthyl 4-carboxylique dans ml de THF anhydre sont ajoutés 1,1 équivalent de triéthylamine et 1, équivalent de diéthylcyanophosphate à 0°C. 1,1 équivalent de n-butylamine sont ajoutés à 0°C et le milieu est agité pendant 1 heure avec un retour à température ambiante. Après évaporation et traitement aqueux par extraction, le brut réactionnel reconcentré est purifié sur colonne de silice (éluant dichlorométhane). Après évaporation des fractions d'intérêt on obtient une huile jaune.

RMN 1 H (DMSO-d6): δ ppm 7,79 (t, NH), 3,53 (d. 2H), 3,1 (d.t, 2H), 2,97 (d, 2H), 1,41 (t.t. 2H), 1,34 (s, 3H), 1,23 (m, 8H), 0,85 (t, 3H) ; MS m/z (M+, 262 ; M+23, 284)
RMN 1 H (DMSO-d6): δ ppm 7,79 (t, NH), 3,54 (d. 2H), 3,08 (d.t, 2H), 2,98 (d, 2H), 1,40 (q 2H), 1,34 (s, 3H), 1,27 (m, 4H), 0,87 (t, 3H) ; ESI+ : [(M, Na) +] = 242 m/z

### Exemple 10: Synthèse du Ethyl 4-methyl-1,2-dithiolane-4-carboxylate (Composé 4)

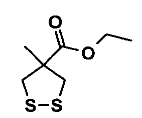

A 1g d'acide 4-methyl-1,2-dithiolane-4-carboxylique dans 20 ml d'éthanol est ajouté de la résine sulfonique DOWEX 50 WX8 (vendue par Aldrich). Le mélange est porté au reflux pendant 24heures puis filtré et évaporé pour conduire à l'ester d'éthyle pur.
RMN 1H (DMSO-d6): δ ppm 4,13 (q, 2H), 3,58 (d, 2H), 3,02 (d, 2H), 1,40 (s,
3H), 1,20 (t, 3H)
ESI+ : [(2M, Na) +] = 407 m/z

### Exemple 11: Synthèse du N-(4-hydroxy-3-methoxybenzyl)-4-methyl-1,2-dithiolane-4-carboxamide (Composé 16)

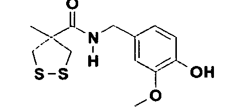

A 24,3 mmol de dithiolane acide, en solution dans 60 ml de dichorométhane, refroidi à 0°C (avec bain de glace) sont ajoutés 24,3 mmol de N-hydroxysuccinimide. Le milieu réactionnel est agité 30 min à 0°C. Une solution de 24,3 mmol de DCC dans 50ml de dichlorométhane est ajouté puis le mélange est agité à 20°C pendant 4 heures. Le milieu réactionnel est filtré et lavé puis le filtrat est évaporé à sec au rotavopor à 40°C sous vide pour conduire au 1-{[(4-methyl-1,2-dithiolan-4-yl)carbonyl]oxy}pyrrolidine-2,5-dione. (m=7g, Rdt quantitatif). A 1,58 mmol de 1-{[(4-methyl-1,2-dithiolan-4-yl)carbonyl]oxy}pyrrolidine-2,5-dione, sont ajoutés 10ml de MethylTHF, 3.16mmol de chlorhydrate de 4-(aminomethyl)-2-methoxyphenol et 3,16 mmol de triéthylamine. Après agitation une nuit, le mélange est filtré, rincé avec du MethylTHF puis évaporé. Une chromatographie flash éluant dichloromethane/ Méthanol 98/2 permet d'obtenir le composé 16 sous forme d'une huile jaune (Rdt 84%)
RMN 1H(DMSO-d6): δ ppm 1,39 (s,3H); 3,03 (d,2H); 3,56 (d, 2H), 3,63 (s, 3H, OCH3), 4,21 (d, 2H), 6,64 (dd, 1 H,Ar), 6,69 (d,1 H, Ar), 6.80 (d, 1 H, Ar), 8,31 (t, 1 H, NH), 8,79 (s,1 H, OH)
ESI+ : [(M, H)+] = 300 m/z

### Exemple 12: Synthèse du N-[2-(4-hydroxy-3-methoxyphenyl)ethyl]-4-methyl-1,2-dithiolane-4-carboxamide (Composé 17)

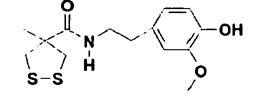

Même méthode que pour le composé 16 avec le chlorhydrate de 4-(2-aminoethyl)-2-methoxyphenol. Huile jaune Rdt=64%
RMN 1H(DMSO-d6): δ ppm 1,31 (s,3H); 2,63 (t, 2H), 2,97(d,2H); 3,24 (m,2H, NCH2) 3,53 (d, 2H), 3,75 (s, 3H, OCH3), 6,57 (dd, 1 H,Ar), 6,67 (d,1H, Ar), 674 (d, 1H, Ar), 8.86(t, 1H, NH), 8,67(s,1H, OH)
ESI+ : [(M, H)+] = 314 m/z

### Exemple 13: Synthèse du N,N-diethyl-4-methyl-1,2-dithiolane-4-carboxamide (Composé 18)

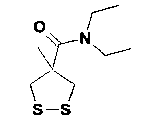

A 2,26 mmol de composé 1 sont ajoutés 5 ml de dichloromethane anhydre, 0,1ml de DMF anhydre. Le mélange est refroidi à 0°C avant l'ajout de 2,7 mmol de chlorure d'oxalyl. Le mélange est agité à 20°C avant d'être ajouté à 0°C à un mélange de 2.26mmol de diéthylamine, 5ml de dichloromethane anhydre, 6.8mmol de Diisopropylethylamine. Le milieu réactionnel est agité 3h à 20°C. Lorsque la réaction est terminée, le milieu est dilué dans 50ml de dichlorométhane, puis lavé avec 2x30ml d'eau, 1x50ml de solution saturée de NH4CI, puis séché sur Na₂SO₄ et évaporé à sec au rotavapor. Après flash chromatographie (éluant heptane/AcOEt) est isolé le composé 18 sous forme d'une huile jaune (Rdt 48%)
RMN 1 H (DMSO-d6): δ ppm 1,36 (s,3H) ; 3,15 (d,2H) ; 3,50 (d, 2H), 3 3 (m,
2x2H), 1,.07(m, 2x3H)
ESI+ : [(M, H)+] = 220 m/z

### Exemple 14: Synthèse du 4-methyl-N-(1-methylethyl)-1,2-dithiolane-4-carboxamide (Composé 20)

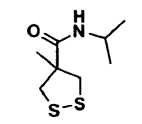

Même méthode que dans l'exemple 19 avec l'isopropylamine.
Solide beige Rdt 54%
RMN 1 H (DMSO-d6): δ ppm 1,06 (d, 2x3H); 1,33 (s,3H, Hc) ; 2,99 (d,2H, Hb) ; 3,56 (d, 2H, Ha); 3,88(m, 1 H),
ESI+ : [(M, H)+] = 206 m/z

### Exemple 15: Synthèse du 4-methyl-N-phenyl-1,2-dithiolane-4-carboxamide (Composé 21)

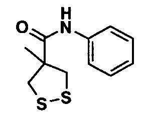

Même méthode que dans l'exemple 19 avec l'aniline. Huile jaune Rdt 70%
RMN 1 H (DMSO-d6): δ ppm 1,51 (s,3H, Hc) ; 2,99 (d,2H, Hb) ; 3,75 (d, 2H, Ha),
7,08 (t, 1 H, Ar), 7,3(t, 2H, Ar), 7.,60(d, 2H, Ar), 9,56(s, 1 h, NH)
ESI+ : [(M, H)+] = 240 m/z

### EXEMPLES DE FORMULATION 16 à 19

| **Compositions** | **Ex16 (*)** | **Ex17** | **Ex18** | **Ex19** | **Ex20** |
|---|---|---|---|---|---|
| Benzoate d'alcools C₁₂/C₁₅ | 10,0 | 10,0 | 10,0 | 10,0 | 10,0 |
| Mélange mono-stéarate de glycéryle/stéarate de PEG (100OE) | 6,5 | 6,5 | 6,5 | 6,5 | 6,5 |
| Polyacrylam ide (and) C13-C14 Isoparaffin (and) Laureth-7 | 1,0 | 1,0 | 1,0 | 1,0 | 1,0 |
| Compose 9 de l'invention | - | 10,0 | - | - | - |
| Composé 10 de l'invention | - | - | 10,0 | - | 10,0 |
| Composé 1 de l'invention | - | - | - | 10,0 | - |
| Composé 2 de l'invention | - | - | - | 10,0 | - |
| 4-Tertiobutyl-4'-Methoxy-Dibenzoylmethane (PARSOL 1789) | 1,0 | 1,0 | 1,0 | 1,0 | 1,0 |
| Glycérine | 5,0 | 5,0 | 5,0 | 5,0 | 5,0 |
| Eau désionisée Eau desionisee | qsp 100 | qsp 100 | qsp 100 | qsp 100 | qsp 100 |

| | | | | | |
|---|---|---|---|---|---|
| (*) hors invention | | | | | |

### Photostabilisation du 4-(ter-butyl) 4'-méthoxy dibenzoylméthane

### PRINCIPE DE LA METHODE

On mesure le pourcentage en perte de dérivé de dibenzoylméthane induite par l'exposition à un simulateur solaire d'une formule étalée en films d'épaisseur voisine de 20µm.

L'évaluation est faite par analyse HPLC du filtre en solution, après extraction des films, en comparant des échantillons irradiés et non irradiés.

### Matériel et conditions mises en oeuvre

Ces émulsions sont étalées en films d'épaisseur voisine de 20µm sur une face dépolie d'un disque de silice. La quantité exacte est déterminée par pesée. surface de 6 quartz dépolis. 3 films sont exposés au simulateur solaire ORIEL 1000W muni d'une sortie 4 pouces, avec filtre 81017 et miroir dichroîque délivrant une dose de 21,6 J/cm² UVA (correspondant à une exposition 1 heure UVA). 3 autres films servent de références. Les échantillons sont exposés en position horizontale.
UV-mètre : appareil OSRAM CENTRA équipé de 2 têtes de lecture, l'une pour l'UVA l'autre pour l'UVB. L'ensemble simulateur-UV mètre est étalonné annuellement par spectroradiométrie.

Mesures d'irradiance effectuées en début et en fin d'exposition en plaçant les têtes de lecture à la position de l'échantillon.

Les irradiances sont : 0.35 - 0.45mW/cm² en UVB et 14-16 mW/cm² en UVA

A la fin de l'exposition chaque disque support est introduit dans un bocal de 600ml avec 10ml d'un solvant approprié (en général EtOH) ; l'ensemble est placé pendant 5 minutes dans une cuve à ultrasons.

La solution est ensuite transférée dans des flacons adaptés au support compatible avec l'appareil d'analyse HPLC utilisé.

Les conditions analytiques peuvent être ajustées en fonction de l'actif testé.

Le taux de Butyl Methoxydibenzoylmethane résiduel est mesuré par Chromatographie : chaîne HPLC avec détecteur à barette de diodes (Waters).

On mesure également le taux résiduel en Butyl Methoxydibenzoylméthane (avobenzone) après irradiation dans le même support ne contenant pas de composé dérivé de dithiolane de formule (I) (Exemple comparatif Formule 1).

Le calcul du taux (%) résiduel est effectué à partir des moyennes obtenues sur les échantillons exposés et non exposés aux UV, comme décrit ci-dessous :

| **Compositions** | **% résiduel d'avobenzone après exposition 1 heure UVA** |
|---|---|
| **Ex 16 (hors invention) (dibenzoylméthane seul)** | 21,0 ± 1,5 |
| **Ex 17: composé 9 + dibenzoylméthane** | • 89,5 ± 1,0 |
| **EX18: composé10 + dibenzoylméthane** | • 87,5 ± 1,7 |
| **Ex 19: composé 1 + dibenzoylméthane** | • 83,1 ± 0,9 |
| **Ex20 : composé 2+ dibenzoylméthane** | • 73,7 ± 1,5 |

## Revendications

1. Composition comprenant dans un support cosmétiquement acceptable au moins un système filtrant UV, **caractérisée par le fait qu'**elle comprend :
(a) au moins un dérivé du dibenzoylméthane et
(b) au moins un composé dithiolane de formule(I) suivante : 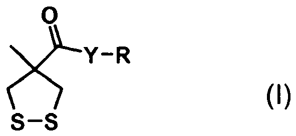 dans laquelle :
Y désigne O ou NR₁
R₁ désigne un atome d'hydrogène ; un groupe hydrocarboné alkyle saturé linéaire en C₁-C₂₀ ou ramifié en C₃-C₂₀ ou insaturé en C₂-C₂₀ ; un groupe aryle éventuellement substitué par un ou plusieurs hydroxyles et/ou par un ou plusieurs radicaux alkoxy en C₁-C₈;
R désigne un atome d'hydrogène ; un groupe hydrocarboné alkyle saturé linéaire en C₁-C₂₀ ou ramifié en C₃-C₂₀ ou insaturé en C₂-C₂₀ ou un groupe aryle ou un groupe alkyle saturé en C₁-C₈ possédant un substituant aryle éventuellement substitué par un ou plusieurs hydroxyles et/ou par un ou plusieurs radicaux alcoxy en C₁-C₈ ;
R possède éventuellement un ou plusieurs substituants choisis parmi OR₂, SR₂, NR₂R₃, COOR₂ dans lesquels :
R₂ désigne un atome d'hydrogène ou un groupe hydrocarboné saturé linéaire en C₁-C₅ ou ramifié en C₃-C₅ ou insaturé en C₂-C₅, ou un groupe aryle
R₃ désigne un atome d'hydrogène ; un groupe hydrocarboné saturé linéaire en C₁-C₅ ou ramifié en C₃-C₅ ou insaturé en C₂-C₅ ; un groupe aryle ; un groupe acétyle.
R et R₁ peuvent former un cycle choisi parmi pyrrolidine, pyrroline, pipéridine, pipérazine, morpholine, thiomorpholine, azépine, ainsi que leurs sels, leurs chélates, leurs solvates et leurs isomères optiques.

2. Composition selon la revendication 1, où le composé de formule (1) est choisi parmi ceux pour lesquels Y est NR₁ et R₁ = H.

3. Composition selon la revendication 1 ou 2, où le composé de formule (I) est choisi parmi ceux pour lesquels R = H ou un radical alkyle en C₁-C₈.

4. Composition selon la revendication 1, où le composé de formule (I), est choisi parmi les composés suivants :
| **N°** | **Structure** | **Nom Chimique** |
|---|---|---|
| 1 | 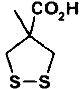 | Acide 4-methyl-1,2-dithiolane-4-carboxylique |
| 2 | 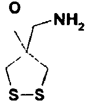 | 4-methyl-1,2-dithiolane-4-carboxamide |
| 3 | 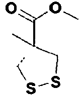 | Méthyl 4-methyl-1,2-dithiolane-4-carboxylate |
| 4 | 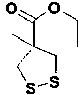 | Ethyl 4-methyl-1,2-dithiolane-4-carboxylate |
| 5 | 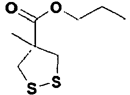 | Propyl 4-methyl-1,2-dithiolane-4-carboxylate |
| 6 | 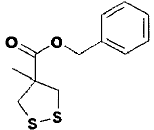 | Benzyl 4-methyl-1,2-dithiolane-4-carboxylate |
| 7 | 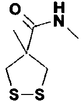 | N-methyl 4-methyl-1,2-dithiolane-4-carboxamide |
| 8 | 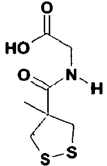 | {[(4-methyl-1,2-dithiolan-4-yl)carbonyl]amino} acetic acide |
| 9 | 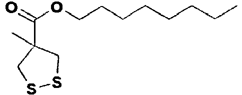 | Octyl 4-methyl-1,2-dithiolane-4-carboxylate |
| 10 | 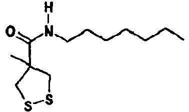 | N-heptyl-4-methyl-1,2-dithiolane-4-carboxamide |
| 11 | 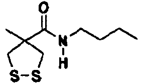 | N-butyl-4-methyl-1,2-dithiolane-4-carboxamide |
| 12 | 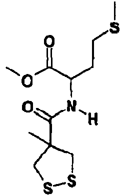 | Methyl 2-{[(4-methyl-1,2-dithiolan-4-yl)carbonyl]amino}-4-(methylsulfanyl)butano ate |
| 14 | 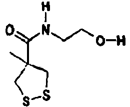 | N-(2-hydroxyethyl)-4-methyl-1,2-dithiolane-4-carboxamide |
| 15 | 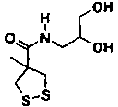 | N-(2,3-dihydroxypropyl)-4-methyl-1,2-dithiolane-4-carboxamide |
| 16 | 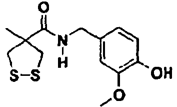 | N-(4-hydroxy-3-methoxybenzyl)-4-methyl-1,2-dithiolane-4-carboxamide |
| 17 | 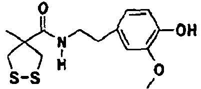 | N-[2-(4-hydroxy-3-methoxyphenyl)ethyl]-4-methyl-1,2-dithiolane-4-carboxamide |
| 18 | 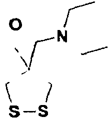 | N,N-diethyl-4-methyl-1,2-dithiolane-4-carboxamide |
| 19 | 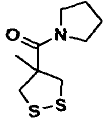 | [(4-methyl-1,2-dithiolan-4-yl)carbonyl]pyrrolidine |
| 20 | 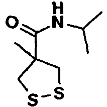 | 4-methyl-N-(1-methylethyl)-1,2-dithiolane-4-carboxamide |
| 21 | 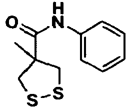 | 4-methyl-N-phenyl-1,2-dithiolane-4-carboxamide |
| 22 | 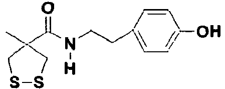 | N-[2-(4-hydroxyphenyl)ethyl]-4-methyl-1,2-dithiolane-4-carboxamide |
| 23 | 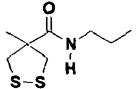 | N-propyl-4-methyl-1,2-dithiolane-4-carboxamide |
| 24 | 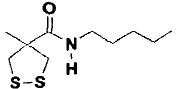 | N-pentyl-4-methyl-1,2-dithiolane-4-carboxamide |
| 25 | 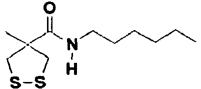 | N-hexyl-4-methyl-1,2-dithiolane-4-carboxamide |

5. Composition selon la revendication 4, où le composé de formule (I), est choisi parmi les composés suivants :
| **N°** | **Structure** | **Nom Chimique** |
|---|---|---|
| 1 | 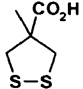 | Acide 4-methyl-1,2-dithiolane-4-carboxylique |
| 2 | 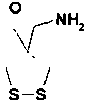 | 4-methyl-1,2-dithiolane-4-carboxamide |
| 9 | 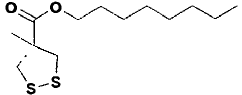 | Octyl 4-methyl-1,2-dithiolane-4-carboxylate |
| 10 | 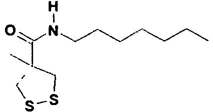 | N-heptyl-4-methyl-1,2-dithiolane-4-carboxamide |

6. Composition selon l'une quelconque des revendications 1 à 5, où le dérivé de dibenzoylméthane est le 4-(ter.-butyl) 4'-méthoxy dibenzoylméthane ou Butyl Methoxy Dibenzoylmethane de formule suivante :

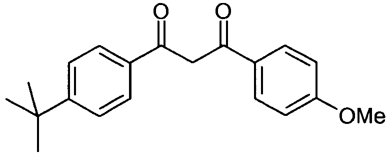

7. Composition selon l'une quelconque des revendications 1 à 6, **caractérisée par le fait qu'**elle contient en plus d'autres filtres organiques ou inorganiques actifs dans l'UV-A et/ou l'UV-B hydrosolubles ou liposolubles ou bien insolubles dans les solvants cosmétiques couramment utilisés.

8. Composition selon la revendication 7, où les filtres organiques complémentaires sont choisis parmi les anthranilates ; les dérivés cinnamiques ; les dérivés salicyliques, les dérivés du camphre ; les dérivés de la benzophénone ; les dérivés de β,β-diphénytacrytate ; les dérivés de triazine ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'a-alkylstyrène ; les 4,4-diarylbutadiènes ; les dérivés de mérocyanines et leurs mélanges.

9. Composition selon la revendication 8, **caractérisée par le fait que** le ou les filtres UV organiques sont choisis parmi les composés suivants :
Ethylhexyl Methoxycinnamate
Ethylhexyl Salicylate,
Homosalate,
Octocrylene,
Phenylbenzimidazole Sulfonic Acide,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Bis-Ethylhexyloxyphenol Methoxyphenyl Triazine
Ethylhexyl triazone,
Diethylhexyl Butamido Triazone,
la 2,4bis (4'-aminobenzoate de n-butyle)-6-(aminopropyltrisiloxane)-s-triazine,
la 2,4,6-tris(4'-amino benzalmalonate de dinéopentyle)-s-triazine
la 2,4,6-tris-(4'-amino benzalmalonate de düsobutyle)-s-triazine
la 2,4bis (4'-aminobenzalmalonate de dinéopentyle)-6-(4'-aminobenzoate de n-butyle)-s-triazine,
la 2,4,6-tris(biphenyl-4-yl-1,3,5-triazine
la 2,4,6-tris(terphenyl)-1,3,5-triazine
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine
Octyl-5-N,N-diethylamino-2-phenysulfonyl-2,4-pentadienoate
et leurs mélanges.

10. Composition selon la revendication 7, **caractérisée par le fait que** les filtres inorganiques complémentaires sont des pigments d'oxydes métalliques, enrobés ou non.

11. Composition selon la revendication 10, où les pigments d'oxydes métalliques enrobés ou non ont une taille moyenne de particule primaire: comprise entre 5 nm et 100 nm et de préférence entre 10 nm et 50 nm.

12. Composition selon la revendication 10 ou 11, où les pigments d'oxydes métalliques enrobés ou non sont choisis parmi des pigments d'oxyde de titane, amorphe ou cristallisé sous forme rutile et/ou anatase, de fer, de zinc, de zirconium ou de cérium ou leurs mélanges.

13. Composition selon l'une quelconque des revendications 1 à 12, **caractérisée par le fait qu'**elle se présente sous la forme d'une émulsion huile-dans-eau ou eau-dans huile.

14. Procédé pour améliorer la stabilité chimique vis-à-vis du rayonnement UV d'au moins un dérivé du dibenzoylméthane tel que défini dans l'une quelconque des revendications précédentes, **caractérisé par le fait qu'**on associe audit dérivé de dibenzoylméthane une quantité efficace d'au moins un composé dithiolane de formule (I) tel que défini dans les revendications précédentes.

15. Utilisation d'au moins un composé dithiolane de formule (I) tel que défini dans les revendications précédentes dans une composition comprenant dans un support cosmétiquement acceptable, au moins un dérivé du dibenzoylméthane tel que défini dans l'une quelconque des revendications précédentes dans le but de d'améliorer l'efficacité de ladite composition vis-à-vis des rayons UV-A.

## Claims

1. Composition comprising, in a cosmetically acceptable support, at least one UV-screening system, **characterized in that** it comprises:
(a) at least one dibenzoylmethane derivative and
(b) at least one dithiolane compound of formula (I) below: 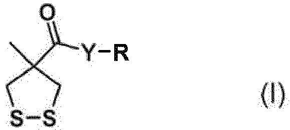 in which:
Y denotes O or NR₁;
R₁ denotes a hydrogen atom; a saturated linear C₁-C₂₀ or branched C₃-C₂₀ or unsaturated C₂-C₂₀ alkyl hydrocarbon-based group; an aryl group optionally substituted with one or more hydroxyls and/or with one or more C₁-C₈ alkoxy radicals;
R denotes a hydrogen atom; a saturated linear C₁-C₂₀ or branched C₃-C₂₀ or unsaturated C₂-C₂₀ alkyl hydrocarbon-based group, an aryl group or a saturated C₁-C₈ alkyl group containing an aryl substituent optionally substituted with one or more hydroxyls and/or with one or more C₁-C₈ alkoxy radicals;
R optionally bears one or more substituents chosen from OR₂, SR₂, NR₂R₃ and COOR₂ in which:
R₂ denotes a hydrogen atom or a saturated linear C₁-C₅ or branched C₃-C₅ or unsaturated C₂-C₅ hydrocarbon-based group, or an aryl group;
R₃ denotes a hydrogen atom; a saturated linear C₁-C₅ or branched C₃-C₅ or unsaturated C₂-C₅ hydrocarbon-based group; an aryl group; an acetyl group;
R and R₁ may form a ring chosen from pyrrolidine, pyrroline, piperidine, piperazine, morpholine, thiomorpholine and azepine,
and also the salts, chelates, solvates and optical isomers thereof.

2. Composition according to Claim 1, in which the compound of formula (I) is chosen from those for which Y is NR₁ and R₁ = H.

3. Composition according to Claim 1 or 2, in which the compound of formula (I) is chosen from those for which R = H or a C₁-C₈ alkyl radical.

4. Composition according to Claim 1, in which the compound of formula (I) is chosen from the following compounds:
| **No.** | **Structure** | **Chemical name** |
|---|---|---|
| 1 | 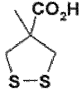 | 4-Methyl-1,2-dithiolane-4-carboxylic acid |
| 2 | 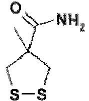 | 4-Methyl-1,2-dithiolane-4-carboxamide |
| 3 | 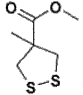 | Methyl 4-methyl-1,2-dithiolane-4-carboxylate |
| 4 | 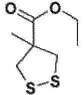 | Ethyl 4-methyl-1,2-dithiolane-4-carboxylate |
| 5 | 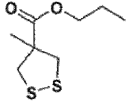 | Propyl 4-methyl-1,2-dithiolane-4-carboxylate |
| 6 | 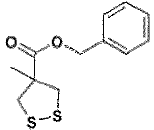 | Benzyl 4-methyl-1,2-dithiolane-4-carboxylate |
| 7 | 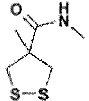 | N-Methyl 4-methyl-1,2-dithiolane-4-carboxamide |
| 8 | 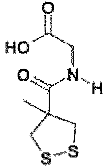 | {[(4-Methyl-1,2-dithiolan-4-yl)carbonyl]amino}acetic acid |
| 9 | 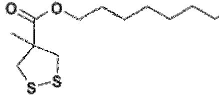 | Octyl 4-methyl-1,2-dithiolane-4-carboxylate |
| 10 | 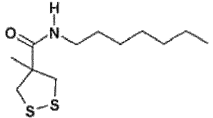 | N-Heptyl-4-methyl-1,2-dithiolane-4-carboxamide |
| 11 | 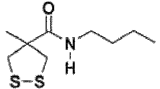 | N-Butyl-4-methyl-1,2-dithiolane-4-carboxamide |
| 12 | 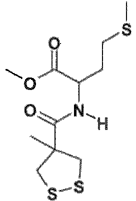 | Methyl 2-{[(4-methyl-1,2-dithiolan-4-yl)carbonyl]-aminol-4-(methylsulfanyl)-butanoate |
| 14 | 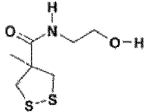 | N-(2-Hydroxyethyl)-4-methyl-1,2-dithiolane-4-carboxamide |
| 15 | 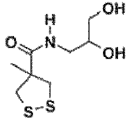 | N-(2,3-Dihydroxypropyl)-4-methyl-1,2-dithiolane-4-carboxamide |
| 16 | 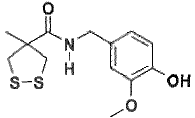 | N-(4-Hydroxy-3-methoxybenzyl)-4-methyl-1,2-dithiolane-4-carboxamide |
| 17 | 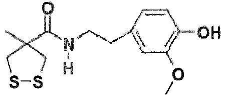 | N-[2-(4-Hydroxy-3-methoxyphenyl)ethyl]-4-methyl-1,2-dithiolane-4-carboxamide |
| 18 | 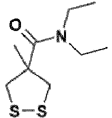 | N,N-Diethyl-4-methyl-1,2-dithiolane-4-carboxamide |
| 19 | 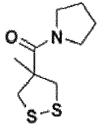 | [(4-Methyl-1,2-dithiolan-4-yl)carbonyl]pyrrolidine |
| 20 | 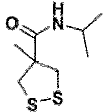 | 4-Methyl-N-(1-methylethyl)-1,2-dithiolane-4-carboxamide |
| 21 | 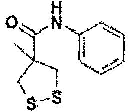 | 4-Methyl-N-phenyl-1,2-dithiolane-4-carboxamide |
| 22 | 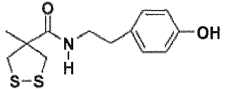 | N-[2-(4-Hydroxyphenyl)-ethyl]-4-methyl-1,2-dithiolane-4-carboxamide |
| 23 | 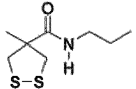 | N-Propyl-4-methyl-1,2-dithiolane-4-carboxamide |
| 24 | 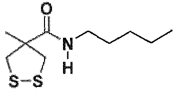 | N-Pentyl-4-methyl-1,2-dithiolane-4-carboxamide |
| 25 | 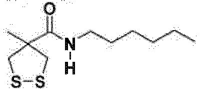 | N-Hexyl-4-methyl-1,2-dithiolane-4-carboxamide |

5. Composition according to Claim 4, in which the compound of formula (I) is chosen from the following compounds:
| **No.** | **Structure** | **Chemical name** |
|---|---|---|
| 1 | 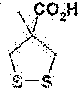 | 4-Methyl-1,2-dithiolane-4-carboxylic acid |
| 2 | 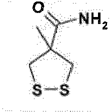 | 4-Methyl-1,2-dithiolane-4-carboxamide |
| 9 | 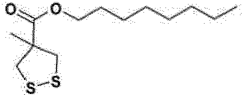 | Octyl 4-methyl-1,2-dithiolane-4-carboxylate |
| 10 | 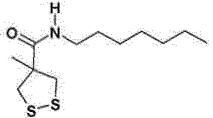 | N-Heptyl-4-methyl-1,2-dithiolane-4-carboxamide |

6. Composition according to any one of Claims 1 to 5, in which the dibenzoylmethane derivative is 4-(tert-butyl)-4'-methoxydibenzoylmethane, or Butyl Methoxy Dibenzoylmethane, of the following formula:

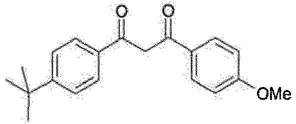

7. Composition according to any one of Claims 1 to 6, **characterized in that** it also contains other UVA-active and/or UVB-active organic or mineral screening agents, which are water-soluble or liposoluble, or else insoluble in the commonly used cosmetic solvents.

8. Composition according to Claim 7, in which the additional organic screening agents are chosen from anthranilates; cinnamic derivatives; salicylic derivatives; camphor derivatives; benzophenone derivatives; β,β-diphenylacrylate derivatives; triazine derivatives; benzotriazole derivatives; benzalmalonate derivatives; benzimidazole derivatives; imidazolines; bis-benzazolyl derivatives; p-aminobenzoic acid (PABA) derivatives; methylenebis(hydroxyphenylbenzotriazole) derivatives; benzoxazole derivatives; screening polymers and screening silicones; α-alkylstyrene-based dimers; 4,4-diarylbutadienes; merocyanin derivatives; and mixtures thereof.

9. Composition according to Claim 8, **characterized in that** the organic UV-screening agent(s) is (are) chosen from the following compounds:
Ethylhexyl methoxycinnamate,
Ethylhexyl salicylate,
Homosalate,
Octocrylene,
Phenylbenzimidazolesulfonic acid,
Benzophenone-3,
Benzophenone-4,
Benzophenone-5,
n-Hexyl 2-(4-diethylamino-2-hydroxybenzoyl)-benzoate,
4-Methylbenzylidenecamphor,
Terephthalylidenedicamphorsulfonic acid,
Disodium phenyldibenzimidazoletetrasulfonate,
Methylenebis(benzotriazolyl)tetramethylbutylphenol,
Bis(ethylhexyloxyphenol)methoxyphenyltriazine, Ethylhexyltriazone,
Diethylhexylbutamidotriazone,
2,4,6-Tris(dineopentyl 4'-aminobenzalmalonate)-s-triazine,
2,4,6-Tris(diisobutyl 4'-aminobenzalmalonate)-s-triazine,
2,4-Bis(n-butyl 4'-aminobenzoate)-6-(aminopropyl-trisiloxane)-s-triazine,
2,4-Bis(dineopentyl 4'-aminobenzalmalonate)-6-(n-butyl 4'-aminobenzoate)-s-triazine,
2,4,6-Tris(biphenyl-4-yl)-1,3,5-triazine,
2,4,6-Tris(terphenyl)-1,3,5-triazine,
Drometrizole trisiloxane,
Polysilicone-15,
1,1-Dicarboxy(2,2'-dimethylpropyl)-4,4-diphenyl-butadiene,
2,4-Bis[5-1(dimethylpropyl)benzoxazol-2-yl(4-phenyl)imino]-6-(2-ethylhexyl)imino-1,3,5-triazine,
Octyl 5-N,N-diethylamino-2-phenylsulfonyl-2,4-pentadienoate,
and mixtures thereof.

10. Composition according to Claim 7, **characterized in that** the additional mineral screening agents are coated or uncoated metal oxide pigments.

11. Composition according to Claim 10, in which the coated or uncoated metal oxide pigments have a mean primary particle size of between 5 nm and 100 nm and preferably between 10 nm and 50 nm.

12. Composition according to Claim 10 or 11, in which the coated or uncoated metal oxide pigments are chosen from titanium oxide (amorphous or crystallized in rutile and/or anatase form), iron oxide, zinc oxide, zirconium oxide or cerium oxide pigments, or mixtures thereof.

13. Composition according to any one of Claims 1 to 12, **characterized in that** it is in the form of an oil-in-water or water-in-oil emulsion.

14. Process for improving the chemical stability, with respect to UV radiation, of at least one dibenzoylmethane derivative as defined in any one of the preceding claims, **characterized in that** the said dibenzoylmethane derivative is combined with an effective amount of at least one dithiolane compound of formula (I) as defined in the preceding claims.

15. Use of at least one dithiolane compound of formula (I) as defined in the preceding claims, in a composition comprising, in a cosmetically acceptable support, at least one dibenzoylmethane derivative as defined in any one of the preceding claims, for the purpose or improving the efficacy of the said composition with respect to UVA rays.

## Patentansprüche

1. Zusammensetzung, die in einem kosmetisch verträglichen Trägermaterial mindestens ein UV filterndes System umfasst, **dadurch gekennzeichnet, dass** sie Folgendes umfasst:
(a) mindestens ein Derivat von Dibenzoylmethan und
(b) mindestens eine Dithiolanverbindung dar folgenden Formel (I): 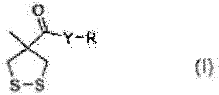 wobei:
Y für O oder NR₁ steht;
R₁ ein Wasserstoffatom bezeichnet, eine gesättigte lineare Alkylkohlenwasserstoffgruppe von C₁-C₂₀, oder eine verzweigte von C₃-C₂₀ oder eine ungesättigte von C₂-C₂₀, eine Arylgruppe von C₁-C₈, die gegebenenfalls durch ein oder mehrere Hydroxyle und/oder durch ein oder mehrere Alkoxyradikal substituiert ist;
R ein Wasserstoffatom bezeichnet, eine gesättigte lineare Alkylkohlenwasserstoffgruppe von C₁-C₂₀, oder eine verzweigte von C₃-C₂₀ oder eine ungesättigte von C₂-C₂₀ oder eine Arylgruppe oder eine gesättigte Alkylgruppe von C₁-C₈, die einen Arylsubstituenten aufweist, der gegebenenfalls durch ein oder mehrere Hydroxyle und/oder durch ein oder mehrere Alkoxyradikale von C₁-C₈ substituiert ist;
R gegebenenfalls einen oder mehrere Substituenten ausgewählt aus OR₂, SR₂, NR₂R₃, COOR₂ aufweist, wobei:
R₂ ein Wasserstoffatom bezeichnet oder eine gesättigte lineare Kohlenwasserstoffgruppe von C₁-C₅, oder eine verzweigte von C₃-C₅ oder eine ungesättigte von C₂-C₅ oder eine Arylgruppe;
R₃ ein Wasserstoffatom bezeichnet, eine gesättigte lineare Kohlenwasserstoffgruppe von C₁-C₅, oder eine verzweigte von C₃-C₅ oder eine ungesättigte von C₂-C₅, eine Arylgruppe, eine Acetylgruppe;
R und R₁ einen Ring bilden können, der ausgewählt ist aus Pyrrolidin, Pyrrolin, Piperidin, Piperazin, Morpholin, Thiomorpholin, Azepin sowie deren Salzen, deren Chelaten, deren Solvaten und deren optischen Isomeren.

2. Zusammensetzung nach Anspruch 1, wobei die Verbindung nach Formel (I) ausgewählt ist aus solchen, bei denen Y NR₁ ist und R₁ = H.

3. Zusammensetzung nach Anspruch 1 oder 2, wobei die Verbindung nach Formel (I) ausgewählt ist aus solchen, bei denen R = H oder ein Alkylradikal von C₁-C₈ ist.

4. Zusammensetzung nach Anspruch 1, wobei die Verbindung der Formel (I) ausgewählt ist aus den folgenden Verbindungen:
| Nr. | Struktur | chemischer Name |
|---|---|---|
| 1 | 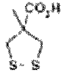 | 4-Methyl-1,2-dithiolan-4-carboxylsäure |
| 2 | 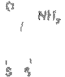 | 4-Methyl-1,2-dithiolan-4-carboxamid |
| 3 | 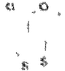 | Methyl-4-methyl-1,2-dithio-lan-4-carboxylat |
| 4 | 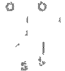 | Ethyl-4-methyl-1,2-dithio-lan-4-carboxylat |
| 5 | 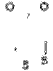 | Propyl-4-methyl-1,2-dithio-lan-4-carboxylat |
| 6 | 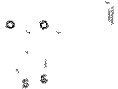 | Benzyl-4-methyl-1,2-dithio-lan-4-carboxylat |
| 7 | 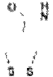 | N-Methyl-4-methyl-1,2-dithiolan-4-carboxamid |
| 8 | 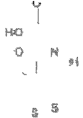 | {[(4-Methyl-1,2-dithiolan-4-yl)carbonyl]amino}essigsäure |
| 9 | 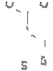 | Octyl-4-methyl-1,2-dithio-lan-4-carboxylat |
| 10 | 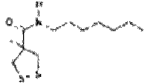 | N-Heptyl-4-methyl-1,2-dithiolan-4-carboxamid |
| 11 | 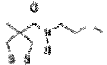 | N-Butyl-4-methyl-1,2-dithio-lan-4-carboxamid |
| 12 | 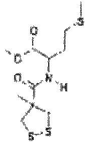 | Methyl-2-{[(4-methyl-1,2-dithiolan-4-yl)carbonyl]amino}-4-(methylsulfanyl)butanoat |
| 14 | 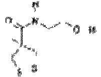 | N-(2-Hydroxyethyl)-4-methyl-1,2-dithiolan-4-carboxamid |
| 15 | 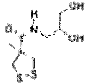 | N-(2,3-Dihydroxypropyl)-4-methyl-1,2-dithiolan-4-carboxamid |
| 16 | 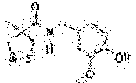 | N-(4-Hydroxy-3-methoxy-benzyl)-4-methyl-1,2-dithio-lan-4-carboxamid |
| 17 | 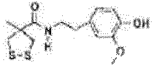 | N-[2-(4-hydroxy-3-methoxy-phenyl)ethyl]-4-methyl-1,2-dithiolan-4-carboxamid |
| 18 | 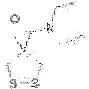 | N,N-Diethyl-4-methyl-1,2-dithiolan-4-carboxamid |
| 19 | 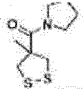 | [(4-Methyl-1,2-dithiolan-4-yl)carbonyl]pyrrolidin |
| 20 | 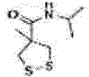 | 4-Methyl-N-(1-methylethyl)-1,2-dithiolan-4-carboxamid |
| 21 | 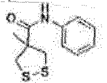 | 4-Methyl-N-phenyl-1,2-dithiolan-4-carboxamid |
| 22 | 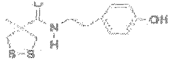 | N-[2-(4-Hydroxy-phenyl)ethyll-4-methyl-1,2-dithiolan-4-carboxamid |
| 23 | 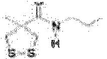 | N-Propyl-4-methyl-1,2-dithiolan-4-carboxamid |
| 24 | 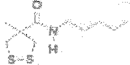 | N-Pentyl-4-methyl-1,2-dithiolan-4-carboxamid |
| 25 | 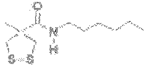 | N-Hexyl-4-methyl-1,2-dithio-lan-4-carboxamid |

5. Zusammensetzung nach Anspruch 4, wobei die Verbindung der Formel (I) ausgewählt ist aus den folgenden Verbindungen:
| Nr. | Struktur | chemischer Name |
|---|---|---|
| 1 | 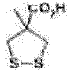 | 4-Methyl-1,2-dithiolan-4-carboxylsäure |
| 2 | 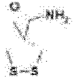 | 4-Methyl-1,2-dithiolan-4-carboxamid |
| 9 | 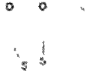 | Octyl-4-methyl-1,2-dithiolan-4-carboxylat |
| 10 | 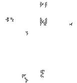 | N-Heptyl-4-methyl-1,2-dithiolan-4-carboxamid |

6. Zusammensetzung nach einem der Ansprüche 1 bis 5, wobei das Dibenzoylmethanderivat das 4-(Ter.-butyl)-4'-methoxy-dibenzoylmethan oder Butylmethoxy-dibenzoylmethan der folgenden Formel ist:

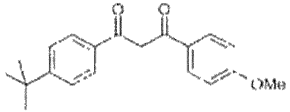

7. Zusammensetzung nach einem der Ansprüche 1 bis 6, **dadurch gekennzeichnet, dass** sie zusätzlich weitere organische oder anorganische Filter aufweist, die im UV-A und/oder UV-B Bereich aktiv, wasserlöslich oder fettlöslich beziehungsweise in häufig verwendeten kosmetischen Lösemitteln unlöslich sind.

8. Zusammensetzung nach Anspruch 7, wobei die zusätzlichen organischen Filter ausgewählt sind unter Anthranilaten; Zimtsäurederivaten; Salicylsäurederivaten; Kampferderivaten; Benzophenonderivaten; β,β-Diphenlyacrlyatderivaten; Triazinderivaten; Benzotriazolderivaten; Benzalmalonatderivaten; Benzimidazolderivaten; Imidazolinen; Bisbenzoazolylderivaten; p-Aminobenzoesäurederivaten (PABA), Methylen-bis-(hydroxyphenyl benzotriazol)derivaten; Benzoxazolderivaten; Polymer- und Silikonfiltern; α-Alkylstyroldimeren; 4,4-Diarylbutadienen; Merocyaninderivaten und Gemischen davon.

9. Zusammensetzung nach Anspruch 8, **dadurch gekennzeichnet, dass** der oder die organischen UV Filter ausgewählt sind aus den folgenden Verbindungen:
Ethylhexyl-methoxycinnamat,
Ethylhexylsalicylat,
Homosalat,
Octocrylen,
Phenylbenzimidazolsulfonsäure,
Benzophenon-3,
Benzophenon-4,
Benzophenon-5,
2-(4-Diethylamino-2-hydroxybenzoyl)-benzoat von N-Hexyl,
4-Methylbenzylidenkampfer,
Terephthalyliden-dikampfer-sulfonsäure,
Dinatriumphenyl-dibenzimidazol-tetrasulfonat,
Methylen-bis-benzotriazolyl-tetramethlbutylphenol,
Bis-ethylhexyloxyphenol-methoxyphenyltriazin,
Ethylhexyltriazon,
Diethylhexylbutamidotriazon,
2,4bis-(N-Butyl-4-aminobenzoat)-6-(aminopropyltrisiloxan)-s-triazin,
2,4,6tris-(Dineopentyl-4'-aminobenzalmalonat)-s-triazin,
2,4,6tris-(Diisobutyl-4'-aminobenzalmalonat)-s-triazin,
2,4bis-(Dineopentyl-4'-aminobenzalmalonat)-6-(N-Butyl-4'-aminobenzoat)-s-triazin,
2,4,6-Tris(biphenyl-4'-yl)-1,3,5-triazin,
2,4,6-Tris(terphenyl)-1,3,5-triazin,
Drometrizoltrisiloxan,
Polysilikon-15,
1,1-Dicarboxy(2,2'-dimethylpropyl)-4,4-diphenylbutadien
2,4-bis-[5-1(Dimethylpropyl)benzoxazol-2-yl-(4-phenyl)-imino]-6-(2-ethylhexyl)-imino-1,3,5-triazin,
Octyl-5-N,N-diethylamino-2-phenylsulfonyl-2,4-pentadienoat
und Gemischen davon.

10. Zusammensetzung nach Anspruch 7, **dadurch gekennzeichnet, dass** die zusätzlichen anorganischen Filter beschichtete oder unbeschichtete Metalloxidpigmente sind.

11. Zusammensetzung nach Anspruch 10, wobei die beschichteten oder unbeschichteten Metalloxidpigmente eine mittlere Größe des Hauptteilchens zwischen 5 nm und 100 nm und vorzugsweise zwischen 10 nm und 50 nm aufweisen.

12. Zusammensetzung nach Anspruch 10 oder 11, wobei die beschichteten oder unbeschichteten Metalloxidpigmente ausgewählt sind aus amorphen oder kristallinen Pigmenten von Titanoxid in Form von Rutil und/oder Anatase, Eisenoxid, Zinkoxid, Zirkoniumoxid oder Ceriumoxid oder Gemischen davon.

13. Zusammensetzung nach einem der Ansprüche 1 bis 12, **dadurch gekennzeichnet, dass** sie in Form einer Ölin-Wasser Emulsion oder einer Wasser-in-Öl Emulsion vorliegt.

14. Verfahren zur Verbesserung der chemischen Stabilität gegenüber UV Strahlung mindestens eines Dibenzoylmethanderivats wie in einem der vorherigen Ansprüche definiert, **dadurch gekennzeichnet, dass** man das Dibenzoylmethanderivat mit einer wirksamen Menge mindestens einer Dithiolanverbindung der Formel (I) wie in den vorherigen Ansprüchen definiert vereinigt.

15. Verwendung mindestens einer Dithiolanverbindung der Formel (I) wie in den vorherigen Ansprüchen definiert in einer Zusammensetzung, die in einem kosmetisch verträglichen Trägermaterial mindestens ein Dibenzoylmethanderivat wie in den vorherigen Ansprüchen definiert umfasst, um die Wirksamkeit der Zusammensetzung gegenüber UV-A Strahlung zu verbessern.
